# FASCICULE DE BREVET EUROPEEN

(11) **EP 2 640 748 B1**
(45) Date de publication et mention de la délivrance du brevet: **08.03.2017**
(21) Numéro de dépôt: 11794861.2
(22) Date de dépôt: 21.11.2011
(51) Int. Cl.: C07K 16/26, A61K 39/395

(54) **LIGAND D'HORMONE LUTEINISANTE ET COMPLEXE LIGAND-GONADOTROPHINE**
LUTEINISIERENDES HORMON-LIGAND UND LIGAND-GONADOTROPHIN-KOMPLEX
LUTEINIZING-HORMONE LIGAND AND LIGAND-GONADOTROPHIN COMPLEX

(30) Priorité: 19.11.2010 FR 1004503
(43) Date de publication de la demande: 25.09.2013
(73) Titulaire: Institut National De La Recherche Agronomique, 75007 Paris (FR)
(72) Inventeur: MAUREL, Marie-Christine, F-37000 Tours (FR); REITER, Eric, F-37380 Nouzilly (FR); GUILLOU, Florian, F-37000 Tours (FR); AUBREY, Nicolas, F-37320 Truyes (FR)
(74) Mandataire: Gevers & Orès
(86) Numéro de dépôt international: PCT/IB2011/055210
(87) Numéro de publication internationale: WO 2012/066519

(56) Documents cités:
- EP-A1- 1 518 863
- EP-A2- 0 137 234
- US-A1- 2002 102 613
- US-A1- 2003 092 082
- CHOPINEAU M ET AL: "Topography of equine chorionic gonadotropin epitopes relative to the luteinizing hormone and follicle-stimulating hormone receptor interaction sites", MOLECULAR AND CELLULAR ENDOCRINOLOGY, ELSEVIER IRELAND LTD, IE, vol. 92, no. 2, 1 avril 1993 (1993-04-01), pages 229-239, XP023489202, ISSN: 0303-7207, DOI: DOI:10.1016/0303-7207(93)90013-A [extrait le 1993-04-01]
- GLENCROSS R G ET AL: "Monoclonal antibody enhancement of FSH-induced uterine growth in snell dwarf mice", JOURNAL OF ENDOCRINOLOGY, SOCIETY FOR ENDOCRINOLOGY, GB, vol. 136, no. 3, 1 mars 1993 (1993-03-01), pages R5-R7, XP009145848, ISSN: 0022-0795
- WEHBI VANESSA ET AL: "Selective modulation of follicle-stimulating hormone signaling pathways with enhancing equine chorionic gonadotropin/antibody immune complexes.", ENDOCRINOLOGY JUN 2010 LNKD- PUBMED:20332198, vol. 151, no. 6, juin 2010 (2010-06), pages 2788-2799, XP009145847, ISSN: 1945-7170

## Description

La présente invention est relative à des anticorps dirigés contre l'hormone lutéinisante (LH), et à leurs utilisations.

L'hormone lutéinisante est une hormone glycoprotéique de la famille des gonadotrophines. Cette famille comprend différentes hormones impliquées dans le fonctionnement des glandes génitales, et la gamétogénèse ; on distingue les gonadotrophines hypophysaires, qui sont produites chez toutes les espèces de mammifères, et qui comprennent outre la LH, l'hormone folliculostimulante (FSH), et les gonadotrophines chorioniques, qui sont produites par le placenta et n'existent que chez certaines espèces de mammifères : l'homme (hCG) et le cheval (eCG).

Les gonadotrophines ont une structure commune : elles sont formées de deux chaînes glycoprotéiques (alpha et beta) associées de manière non-covalente. Au sein d'une même espèce, la chaîne alpha est commune à la LH, à la FSH, à la TSH et le cas échéant à la gonadotrophine chorionique, et c'est la chaîne beta qui est responsable de la spécificité de l'hormone.

Chez les femelles, la FSH est responsable de la croissance et de la différenciation des ovocytes et la LH induit la croissance terminale des ovocytes et l'ovulation. Chez les mâles, la LH stimule la production de testostérone.

La LH comme la FSH sont, chez les femelles, à des concentrations plasmatiques très faibles en période de repos sexuel et en dehors de la période ovulatoire. En période préovulatoire intervient un pic de sécrétion de ces hormones, qui conduit au déclenchement de l'ovulation.

Les gonadotrophines sont utilisées en médecine humaine et vétérinaire pour mimer les mécanismes endocriniens gouvernant les cycles sexuels. Par exemple, dans les élevages ovins et caprins, on utilise l'eCG (qui, chez les espèces autres que l'espèce équine, possède une double activité LH et FSH) associée à un progestogène, pour induire et synchroniser l'oestrus et l'ovulation, ainsi que dans le cadre de traitements de superovulation.

Bien que ces techniques aient démontré leur efficacité, elles présentent un risque sanitaire non négligeable dans la mesure où l'eCG est extraite de plasmas de juments gestantes. En outre, chez certains animaux, l'utilisation répétée d'eCG induit une réponse immunitaire qui se traduit par la sécrétion d'anticorps anti-eCG dans le plasma. Dans la plupart des cas, cette réponse immunitaire aboutit à la neutralisation de l'activité de l'eCG, se traduisant par une baisse d'efficacité du traitement. Chez un petit nombre d'animaux, il a toutefois été constaté que les anticorps anti-eCG produits avaient au contraire la propriété de potentialiser la bioactivité de l'eCG. Des anticorps polyclonaux possédant cette propriété sont décrits dans la Demande EP1518863. Ces anticorps nécessitent toutefois l'administration simultanée d'eCG.

Les Inventeurs ont maintenant obtenu des anticorps monoclonaux produits contre la LH endogène ovine, et ont observé que certains de ces anticorps étaient capables de potentialiser son action.

Ces anticorps monoclonaux potentialisants sont respectivement dénommés ci-après 9A4 A7 D3 (également désigné ci-après D3), 1A6 C4 G11 (également désigné ci-après G11) et 9A4 D4 B6 (également désigné ci-après B6).

L'hybridome produisant l'anticorps 9A4 A7 D3 a été déposé, selon le Traité de Budapest, le 30 juin 2010 auprès de la CNCM (Collection Nationale de Culture de Microorganismes Institut Pasteur, 25 rue du Docteur Roux, 75724 Paris Cedex 15, France), sous le numéro CNCM I-4333.

L'hybridome produisant l'anticorps 1A6 C4 G11 a été déposé, selon le Traité de Budapest, le 30 juin 2010 auprès de la CNCM, sous le numéro CNCM I-4332.

L'hybridome produisant l'anticorps 9A4 D4 B6 a été déposé, selon le Traité de Budapest, le 30 juin 2010 auprès de la CNCM, sous le numéro CNCM I-4334.

En outre, les Inventeurs ont découvert que, de façon surprenante, deux de ces anticorps, à savoir 9A4 A7 D3 et 9A4 D4 B6, possédaient également un effet potentialisant de la FSH.

Les séquences des régions variables de la chaîne lourde et de la chaîne légère de ces anticorps ont été déterminées. Ces séquences, ainsi que les séquences polypeptidiques déduites, sont représentées dans le Tableau I ci-après (pour la chaîne légère et la chaîne lourde de 9A4 A7 D3), dans le Tableau II ci-après (pour la chaine légère et la chaîne lourde de 1A6 C4 G11) et dans le Tableau III ci-après (pour la chaîne légère et la chaîne lourde de 9A4 D4 B6). Les séquences nucléotidiques sont également représentées dans la liste de séquences en annexe respectivement sous les numéros SEQ ID NO:1, 3, 5, 7, 9 et 11, et les séquences polypeptidiques sont également représentées respectivement sous les numéros SEQ ID NO:2, 4, 6, 8, 10 et 12.

**Tableau I**

| Anticorps 9A4 A7 D3 | |
|---|---|
| Chaîne lourde (VH) | |
| Séquence nucléotidique SEQ ID NO :1 | |
| Séquence peptidique SEQ ID NO :2 | |

| Chaîne légère (VL) | |
|---|---|
| Séquence nucléotidique SEQ ID NO :3 | |
| Séquence peptidique SEQ ID NO :4 | |

**Tableau II**

| Anticorps 1A6 C4 G11 | |
|---|---|
| Chaîne lourde (VH) | |
| Séquence nucléotidique SEQ ID NO :5 | |
| Séquence peptidique SEQ ID NO :6 | |

| Chaîne légère (VL) | |
|---|---|
| Séquence nucléotidique SEQ ID NO :7 | |
| Séquence peptidique SEQ ID NO :8 | |

**Tableau III**

| Anticorps 9A4 D4 B6 | |
|---|---|
| Chaîne lourde (VH) | |
| Séquence nucléotidique SEQ ID NO :9 | |
| Séquence peptidique SEQ ID NO :10 | |

| Chaîne légère (VL) | |
|---|---|
| Séquence nucléotidique SEQ ID NO :11 | |
| Séquence peptidique SEQ ID NO :12 | |

Les séquences codant les CDRs de 9A4 A7 D3, de 1A6 C4 G11 et de 9A4 D4 B6 ont également été déterminées, à partir des séquences des chaînes lourdes et des chaînes légères ci-dessus, à l'aide du logiciel IMGT/V-QUEST (Giudicelli et al., Nucleic Acids Research 32, W435-W440, 2004 ; Brochet, X. et al., Nucl. Acids Res. 36, W503-508, 2008). Ces séquences sont identiques pour les trois anticorps.

Les séquences polypeptidiques déduites sont représentées ci-après dans le Tableau IV pour les trois anticorps 9A4 A7 D3, 1A6 C4 G11 et 9A4 D4 B6. Elles sont également représentées dans la liste de séquences en annexe sous les numéros SEQ ID NO: 13 à 17.

**Tableau IV**

| Références | Séquence polypeptidique |
|---|---|
| Chaîne lourde | |
| VH-CDR1 (SEQ ID NO :13) | GYTFTNYW |
| VH-CDR2 (SEQ ID NO :14) | IYPGGGYT |
| VH-CDR3 (SEQ ID NO :15) | ARTPLYGSSYGGFAY |

| Chaîne légère | |
|---|---|
| VL-CDR1 (SEQ ID NO :16) | QGISNY |
| VL-CDR2 | YTS |
| VL-CDR3 (SEQ ID NO :17) | QQYSKLPWT |

La présente invention a pour objet un ligand d'une hormone lutéinisante (LH), caractérisé en ce qu'il comprend le paratope d'un anticorps anti-LH ovine dont le domaine variable de la chaîne lourde contient les CDRs suivants :
- VH-CDR1, défini par la séquence GYTFTNYW (SEQ ID NO: 13);
- VH-CDR2, défini par la séquence IYPGGGYT (SEQ ID NO: 14);
- VH-CDR3, défini par la séquence ARTPLYGSSYGGFAY (SEQ ID NO: 15); et
le domaine variable de la chaîne légère contient les CDRs suivants :
- VL-CDR1, défini par la séquence QGISNY (SEQ ID NO: 16);
- VL-CDR2, défini par la séquence YTS;
- VL-CDR3, défini par la séquence QQYSKLPWT (SEQ ID NO: 17).

Les CDRs (régions déterminant la complémentarité) sont les portions des régions variables d'un anticorps impliquées dans la spécificité de reconnaissance de l'antigène.

On définit ici comme « anticorps anti-LH ovine » tout anticorps obtenu par immunisation d'un animal avec de la LH ovine, et capable de se lier avec celle-ci. Cette définition n'est pas limitée à des anticorps capables de se lier sélectivement avec la LH ovine, mais englobe aussi des anticorps capables de se lier également avec des LH d'autres mammifères, par exemple la LH bovine, caprine, porcine ou humaine ; de même, elle englobe aussi des anticorps capables de se lier également avec une ou plusieurs autre(s) gonadotrophine(s) (ovine(s) ou d'une autre origine), telles que notamment la FSH, ou la hCG. De même, le terme « ligand de LH », englobe aussi des ligands capables de se lier également avec une ou plusieurs autre(s) gonadotrophine(s).

On définit ici comme « gonadotrophine » toute protéine à activité gonadotrope, c'est-à-dire capable de stimuler les récepteurs FSH et LH, qu'il s'agisse d'une protéine naturelle ou recombinante. Plus particulièrement, les termes « LH » et « FSH » englobent, outre les LH ou FSH naturelles, des LH ou FSH recombinantes éventuellement modifiées pour optimiser leurs propriétés pharmacologiques. A titre d'exemple non-limitatif, on citera la corifollitropine alfa, qui est une FSH chimérique résultant de la fusion du peptide carboxy-terminal de la sous-unité bêta de la gonadotrophine chorionique humaine (hCG) avec la chaîne bêta de la FSH humaine, ce qui a pour effet de prolonger sa demi-vie, et en conséquence sa durée d'action, sans affecter son activité FSH.

Les Inventeurs ont testé les capacités de liaison des trois anticorps anti-LH ovine 9A4 A7 D3, 9A4 D4 B6, et 1A6 C4 G11 avec la LH ovine, bovine, ou porcine, la FSH ovine, bovine, porcine, ou humaine, ainsi que la hCG, et ont constaté qu'ils étaient capables de se lier à toutes ces gonadotrophines.

Les séquences des CDR1, CDR2 et CDR3 de la chaîne légère, ainsi que les séquences des CDR1, CDR2 et CDR3 de la chaîne lourde sont identiques pour les trois anticorps monoclonaux B6, D3 et G11. De même, les séquences des frameworks FR2, FR3 et FR4 de la chaîne légère, ainsi que les séquences des FR2, FR3 et FR4 de la chaîne lourde sont identiques.

En revanche, les séquences N-terminales des FR1 des chaînes VH et VL varient selon l'anticorps concerné :
Ainsi, dans le cas de l'anticorps G11 (qui se lie à la LH et à la FSH, mais ne potentialise que la LH) la séquence N-terminale déterminée pour la région FR1 de la chaîne légère est DIQMTQTTSS (SEQ ID NO : 18), et celle déterminée pour la région FR1 de la chaîne lourde est EVKLQQSGAE (SEQ ID NO : 19).

Dans le cas de l'anticorps B6 (qui se lie à la LH et à la FSH, et potentialise ces deux gonadotrophines), la séquence N-terminale déterminée pour la région FR1 de la chaîne légère est DIVMTQATSS (SEQ ID NO : 20), et celle déterminée pour la région FR1 de la chaîne lourde est EVQLQQSGAE (SEQ ID NO : 21).

Dans le cas de l'anticorps D3 (qui se lie à la LH et à la FSH, et potentialise ces deux gonadotrophines), la séquence N-terminale déterminée pour la région FR1 de la chaîne légère est KTQTTSS (SEQ ID NO : 22), et celle déterminée pour la région FR1 de la chaîne lourde est EVQLQESGAE (SEQ ID NO :23).

Dans le cas du scFv B6, qui est dérivé de l'anticorps B6 et qui possède les mêmes propriétés de liaison à la LH et à la FSH et de potentialisation de ces deux gonadotrophines que les anticorps B6 et D3, la séquence N-terminale de la région FR1 du domaine VH diffère de la SEQ ID NO : 21 par la présence d'une glutamine N-terminale, au lieu d'un acide glutamique.

Sans que cette hypothèse soit limitative, la capacité à potentialiser la FSH semble déterminée par la séquence N-terminale de la région FR1 de la chaîne lourde, et notamment par la nature de l'acide aminé en position 3. La présence d'une lysine à cette position paraît associée à la capacité de l'anticorps à potentialiser uniquement l'activité de la LH, alors que la présence d'une glutamine paraît associée à la capacité de l'anticorps à potentialiser à la fois l'activité de la LH et celle de la FSH.

Selon un premier mode de réalisation d'un ligand de LH ledit ligand potentialise la LH mais non la FSH, et la portion N-terminale de la région framework FR1 de sa chaine lourde est définie par la séquence EVKLQQSGAE (SEQ ID NO : 19).

Selon un second mode de réalisation d'un ligand de LH ledit ligand potentialise la LH et la FSH, et la portion N-terminale de la région framework FR1 de sa chaine lourde est définie par la séquence X₁VQLQX₁SGAE (SEQ ID NO : 24) dans laquelle X₁ représente une glutamine ou un acide glutamique, de préférence une glutamine.

Selon une disposition préférée de l'un ou l'autre de ces modes de réalisation, la portion N-terminale de la région FR1 de la chaine légère dudit ligand contient la séquence X₂TQX₃TSS (SEQ ID NO : 25), dans laquelle X₂ représente une méthionine ou une lysine et X₃ représente une thréonine ou une alanine ; avantageusement, ladite portion N-terminale est définie par la séquence DIX₄X₂TQX₃TSS (SEQ ID NO : 26), dans laquelle X₂ et X₃ sont tels que définis ci-dessus, et X₄ représente une glutamine ou une valine.

A titre d'exemples, ladite portion N-terminale peut être définie par l'une des séquences suivantes :
- la séquence DIQMTQTTSS (SEQ ID NO : 18) ;
- la séquence DIVMTQATSS (SEQ ID NO : 20) ;
- la séquence DIQMTQATSS (SEQ ID NO: 27) ;
- la séquence KTQTTSS (SEQ ID NO : 22).

Un ligand de LH conforme au premier mode de réalisation est par exemple un ligand contenant la région FR1 de la chaîne légère et la région FR1 de la chaîne lourde de l'anticorps G11, et avantageusement, la totalité des domaines variables VH et VL dudit anticorps.

Des ligands de LH conformes au second mode de réalisation sont par exemple :
- un ligand contenant la région FR1 de la chaîne légère et la région FR1 de la chaîne lourde de l'anticorps D3, et avantageusement, la totalité des domaines variables VH et VL dudit anticorps ;
- un ligand contenant la région FR1 de la chaîne légère et la région FR1 de la chaîne lourde de l'anticorps B6, et avantageusement, la totalité des domaines variables VH et VL dudit anticorps ;
- un ligand contenant la région FR1 du domaine variable VH et la région FR1 du domaine variable VL du fragment scFv B6, et avantageusement la totalité des domaines variables VH et VL dudit fragment scFv.

Des ligands de LH conformes à l'invention englobent notamment :
a) l'anticorps monoclonal 1A6 C4 G11 produit par l'hybridome CNCM I-4332 ;
b) l'anticorps monoclonal 9A4 A7 D3 produit par l'hybridome CNCM I-4333 ;
c) l'anticorps monoclonal 9A4 D4 B6 produit par l'hybridome CNCM I-4334 ;
d) un fragment Fab, Fab', Fab'2 d'un anticorps a), b) ou c) ci-dessus ;
e) une protéine recombinante comprenant le paratope d'un anticorps a), b) ou c) ci-dessus.

Les protéines recombinantes conformes à l'invention peuvent être notamment des anticorps recombinants dérivés des anticorps monoclonaux a), b) ou c) ci-dessus, modifiés afin de réduire leur immunogénicité chez l'animal ou l'homme auquel ils sont destinés à être administrés.

Un anticorps recombinant conforme à l'invention peut par exemple être un anticorps chimérique, c'est-à-dire un anticorps recombinant conservant les domaines variables de l'anticorps monoclonal dont il est issu, mais dont les domaines constants ont été substitués par ceux d'un autre anticorps, généralement ceux d'un anticorps originaire de l'espèce à laquelle appartient le sujet auquel l'anticorps chimérique doit être administré.

Il peut s'agir également d'un anticorps recombinant dans lequel le paratope de l'anticorps monoclonal murin d'origine (dénommé anticorps « donneur »), est transféré dans un anticorps (dénommé anticorps « accepteur ») originaire de l'espèce à laquelle appartient le sujet auquel l'anticorps recombinant doit être administré, en remplacement du paratope dudit anticorps accepteur. Un tel anticorps recombinant est dénommé « anticorps humanisé » si l'anticorps accepteur est un anticorps humain. Si l'anticorps accepteur est, par exemple, d'origine ovine, caprine, bovine, porcine, etc..., les anticorps recombinants correspondants sont respectivement dénommés « anticorps ovinisé », « anticorps caprinisé », « anticorps bovinisé », « anticorps porcinisé », etc...

Différentes méthodes pour effectuer ce remplacement sont connues en elles-mêmes. Les plus usitées sont basées sur la greffe de CDRs, qui consiste à remplacer les CDRs de l'anticorps accepteur par ceux de l'anticorps donneur. Dans certains cas, la greffe de CDRs peut être complétée par l'optimisation des régions FR, qui consiste à insérer des acides aminés des régions FR de l'anticorps donneur impliqués dans ses propriétés de liaison à l'antigène à la place des acides aminés correspondants des régions FR de l'anticorps accepteur, afin d'optimiser les propriétés de liaison à l'antigène de l'anticorps recombinant final (cf. par exemple ROUTLEDGE et al., "Reshaping antibodies for therapy", in Protein Engineering of Antibody Molecules for Prophylatic and Therapeutic Applications in Man, 13-44, Academic Titles, Nottingham, England, 1993, ou ROGUSKA et al., Protein Engineering, 9(10): 895-904, 1996).

Des anticorps recombinants conformes à l'invention, sont de préférence des immunoglobulines de classe IgM, et notamment d'isotype Kappa.

Des anticorps recombinantes conformes à l'invention peuvent également être des fragments d'anticorps comprenant le paratope d'un anticorps a), b), ou c) ci-dessus. Il peut s'agir notamment de fragments Fab, Fab', Fab'2, Fv, dsFv, ou scFv, ou encore de diabodies, triabodies ou tetrabodies.

Les fragments monovalents Fab contiennent chacun une chaîne légère et la première moitié d'une chaîne lourde reliées entre elles par un pont disulfure. Les fragments bivalents Fab'2 comprennent deux fragments Fab et une partie de la région charnière. Les fragments monovalents Fab' résultent de la coupure du pont disulfure dans la région charnière des fragments Fab'2.

Les fragments Fv sont constitués des domaines variables des chaînes VH et VL d'un anticorps, associés l'un à l'autre par des interactions hydrophobes. Le fragment dsFv est constitué d'un dimère VH::VL relié par un pont disulfure. Les fragments scFv sont constitués des portions variables des chaînes lourdes et légères d'un anticorps, reliées entre elles par l'intermédiaire d'un peptide lieur flexible (Clackson et al., Nature, 352: 624-628, 1991), formant ainsi une protéine simple-chaîne. Les fragments Fv, dsFv, et scFv sont monovalents. Les diabodies, triabodies et tétrabodies sont des formes bi-, tri-, ou tétravalentes, résultant de la multimérisation de respectivement deux, trois, ou quatre fragments scFv.

Le cas échéant, ces fragments d'anticorps peuvent être associés avec des molécules permettant de prolonger leur demi-vie plasmatique lors de leur administration *in vivo,* ils peuvent par exemple être fusionnés avec un polypeptide hydrosoluble de masse moléculaire suffisante pour que la masse moléculaire du polypeptide de fusion ainsi obtenu soit supérieure au seuil de filtration rénale, ou bien conjugués avec un polyol, par exemple le polyéthylène glycol.

Selon un mode de réalisation préféré d'un anticorps de la LH ovine conforme à l'invention, il s'agit d'un fragment scFv.

A titre d'exemple non limitatif, la séquence peptidique d'un fragment scFv conforme à l'invention, dérivé de l'anticorps CNCM I-4334, est représentée dans la liste de séquences en annexe sous le numéro SEQ ID NO: 28.

Des fragments Fab et Fab'2 peuvent être obtenus à partir d'un anticorps conforme à l'invention, par digestion enzymatique, par la papaïne dans le cas d'un fragment Fab, et par la pepsine dans le cas d'un fragment Fab'2. Les fragments Fab' peuvent être obtenus à partir des fragments Fab'2 par coupure du pont disulfure dans la région charnière.

Ces fragments peuvent aussi être obtenus, de même que les anticorps recombinants, les fragments Fv, dsFv, scFv et leurs dérivés multivalents, par les techniques classiques du génie génétique, telles que celles décrites par SAMBROOK et al. (MOLECULAR CLONING, A LABORATORY MANUAL, 2nd Ed., Cold Spring Harbor Laboratory Press, Cold Spring Harbor, N.Y., 1989).

Des polynucléotides codant les régions variables des anticorps monoclonaux 9A4 A7 D3, 1A6 C4 G11 et 9A4 D4 B6 peuvent être obtenus par clonage desdites régions à partir de banques d'ADNc des hybridomes CNCM I-4333, CNCM I-4332 et CNCM I-4334. Ils peuvent également être préparés totalement ou partiellement par synthèse d'acides nucléiques, à partir des séquences nucléotidiques desdites régions variables.

La présente invention a également pour objet toute molécule d'acide nucléique codant un ligand conforme à l'invention, ainsi que tout vecteur recombinant, notamment tout vecteur d'expression, comprenant ladite molécule d'acide nucléique.

Des molécules d'acide nucléique peuvent avantageusement comprendre, outre une séquence codant une protéine recombinante conforme à l'invention, une séquence codant un peptide signal permettant la sécrétion de ladite protéine ; elles peuvent aussi comprendre une ou plusieurs séquence(s) codant un ou plusieurs peptide(s) marqueur(s) permettant la détection et/ou facilitant la purification de ladite protéine.

Des vecteurs d'expression comprennent au moins une séquence d'acide nucléique codant une protéine conforme à l'invention, associée à des éléments de contrôle de la transcription et de la traduction actifs dans la cellule-hôte choisie. Des vecteurs-hôtes utilisables pour la construction de vecteurs d'expression conformes à l'invention sont connus en eux-mêmes, et seront choisis notamment en fonction de la cellule-hôte que l'on souhaite utiliser.

Le présent document a également pour objet toute cellule exprimant un ligand de la LH ovine conforme à l'invention. Ceci englobe notamment les hybridomes CNCM I-4333, CNCM I-4332 et CNCM I-4334, ainsi que les cellules-hôtes transformées par une molécule d'acide nucléique conforme à l'invention.

Des cellules-hôtes utilisables peuvent être des cellules procaryotes ou eucaryotes. La construction de vecteurs d'expression conformes à l'invention et la transformation des cellules-hôtes peuvent être effectuées par les techniques classiques de biologie moléculaire.

Ce document a également pour objet un procédé de production d'un ligand de LH conforme à l'invention, caractérisé en ce qu'il comprend la mise en culture d'au moins une cellule conforme à l'invention, et la récupération dudit ligand à partir de ladite culture.

Si le ligand est sécrété, il peut être récupéré directement à partir du milieu de culture ; sinon, on procédera préalablement à la lyse des cellules ou à la récupération du périplasme dans le cas d'une expression dans *Escherichia coli.*

Le ligand peut ensuite être purifié à partir du liquide d'ascite, du milieu de culture ou du lysat cellulaire par des procédures classiques, connues en elles-mêmes de l'homme de l'art, par exemple par précipitation fractionnée, notamment précipitation au sulfate d'ammonium, électrophorèse, filtration sur gel, chromatographie d'affinité, chromatographie d'échange d'ions etc.

Les ligands selon l'invention sont utilisables dans tous les cas où l'on souhaite potentialiser l'activité LH, non seulement de la LH ovine mais de manière plus générale de toute LH naturelle ou recombinante reconnue par lesdits ligands. Les ligands contenant le paratope des anticorps D3 et B6, permettent en outre de potentialiser l'activité FSH de toute FSH naturelle ou recombinante reconnue par ledit ligand.

Ce document décrit aussi un complexe formé par un ligand selon l'invention et une gonadotrophine capable de se lier audit ligand, et notamment une gonadotrophine, naturelle ou recombinante, dont l'action est potentialisée par ledit ligand.

Des complexes conformes à l'invention englobent notamment :
- un complexe d'un ligand conforme à l'invention avec la LH ;
- un complexe d'un ligand conforme à l'invention avec la hCG ;
- un complexe d'un ligand conforme à l'invention, dérivé de l'un des anticorps D3 ou B6, avec la FSH.

Les complexes peuvent être obtenus par simple incubation d'un ligand conforme à l'invention avec la gonadotrophine choisie. L'administration d'un complexe conforme à l'invention permet d'amplifier l'activité de la gonadotrophine complexée, et par conséquent de diminuer à la fois la dose de gonadotrophine à injecter et le nombre d'injections à faire pour aboutir à une même réponse physiologique, voire à une meilleure réponse, par rapport à la même gonadotrophine utilisée seule.

Les ligands ou les complexes ligand-gonadotrophine peuvent être utilisés *in vitro* pour analyser la potentialisation de la bioactivité des dites gonadotrophines au niveau de leur(s) récepteur(s) cible(s). Ils peuvent être utilisés comme outils d'investigation pour étudier les modifications induites par le phénomène de potentialisation sur l'activation des voies de signalisation des récepteurs cibles, sur l'internalisation de ces récepteurs et sur leur désensibilisation.

Ils peuvent également être utilisés *in vivo* notamment comme médicament, pour augmenter la bioactivité de la LH, ou, dans le cas des ligands dérivés des anticorps B6 et D3, pour augmenter à la fois la bioactivité de la LH et celle de la FSH.

Pour cela, ils sont utilisés soit pour amplifier l'activité LH ou l'activité LH et FSH endogène chez le sujet à traiter, soit pour amplifier l'activité d'une LH ou FSH exogène. Dans le premier cas, le complexe ligand-gonadotrophine endogène se forme après l'injection du ligand chez le sujet à traiter, l'anticorps jouant le rôle de substitut non-hormonal. Dans le deuxième cas, le ligand joue le rôle de potentialisant de la gonadotrophine injectée ; la gonadotrophine et le ligand peuvent être soit préalablement mélangés pour former un complexe ligand-gonadotrophine préalablement à l'administration, soit administrés séparément.

Les ligands ou les complexes ligand-gonadotrophine peuvent être utilisés en particulier :
- pour induire l'ovulation chez un mammifère femelle. Leur administration peut permettre notamment de mimer le pic de sécrétion de LH et, si on le souhaite, de FSH, qui survient normalement en période pré-ovulatoire, et ainsi de déclencher une ovulation ;
- dans le cadre du traitement d'états pathologiques résultants de faibles niveaux circulants de LH et FSH, tels que par exemple des pathologies résultant d'une insuffisance hypophysaire ;
- pour traiter des sujets, mâles ou femelles, présentant une hyporéceptivité des gonades à la LH et la FSH.

Les ligands ou les complexes ligand-gonadotrophine peuvent être utilisés chez l'homme ou chez différents mammifères, notamment des animaux d'élevage (par exemple ovins, bovins, caprins, porcins, équins), ou des animaux de compagnie (par exemple chiens ou chats).

Les ligands ou les complexes ligand-gonadotrophine seront administrés de préférence par injection, celle-ci peut se faire indifféremment par voie intramusculaire, intraveineuse, intra-péritonéale, intradermique, intra-orbitaire ou sous-cutanée sans altérer l'effet potentialisant du ligand.

La présente invention sera mieux comprise à l'aide du complément de description qui va suivre, qui se réfère à des exemples non limitatifs de préparation et d'utilisation de anticorps de LH conformes à l'invention.

### EXEMPLE 1 : MESURE IN VITRO DE L'EFFET POTENTIALISANT DES ACM ET CARACTÉRISATION DES ACM POTENTIALISANTS

Les effets potentialisants des anticorps monoclonaux (AcM) sécrétés par les hybridomes CNCM I-4333, I-4332 et I-4334 ont été mesurés par un dosage biologique *in vitro* spécifique de l'activité LH, réalisé avec la lignée cellulaire MLTC qui exprime de façon stable le récepteur LH. La réponse mesurée est la sécrétion en AMPc après 3 heures de stimulation à 37°C avec la LH seule ou préalablement incubée avec le surnageant des hybridomes.

En outre, l'effet potentialisant de l'activité FSH a été mesuré par un dosage biologique *in vitro,* réalisé avec la lignée cellulaire LTK (lignée de fibroblastes de souris) qui exprime de façon stable le récepteur FSH, ou sur cellules de granulosa bovines en suspension. La réponse mesurée est la sécrétion en AMPc après 3 heures de stimulation à 37°C avec la FSH seule ou préalablement incubée avec le surnageant des hybridomes.

La comparaison de la réponse biologique obtenue en présence de l'hormone seule et celle obtenue avec l'hormone préalablement incubée avec un surnageant d'hybridome, préalablement dosé en anticorps anti-LH, permet de déterminer si celui-ci exerce un effet potentialisant, un effet inhibiteur ou aucun effet.

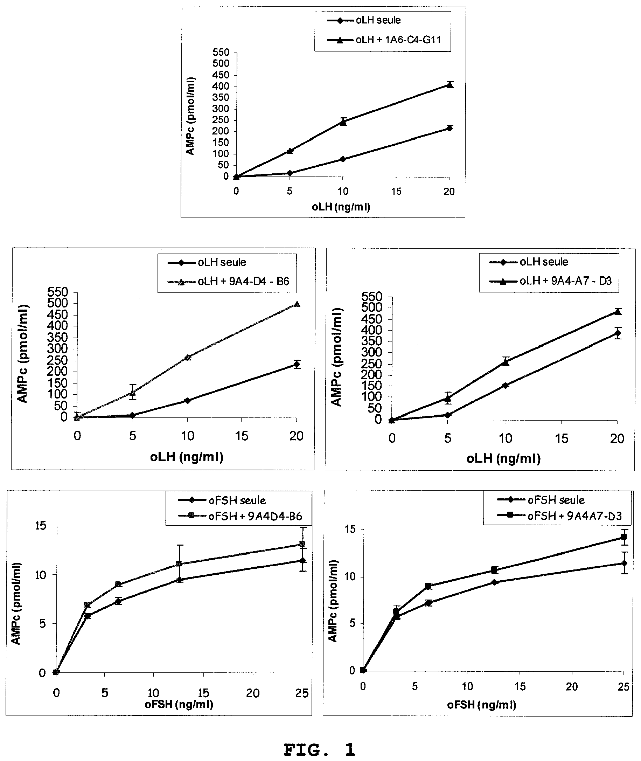

Les résultats du dosage biologique sont illustrés à la Figure 1.

Parmi les 3 hybridomes, un (I-4332) est sécréteur d'anticorps potentialisant l'activité de la oLH strictement (1A6-C4-G11), l'effet potentialisant allant de 700 à 1300% pour le point 5 ng/ml d'hormone. De façon remarquable, les deux autres hybridomes sont sécréteurs d'anticorps potentialisant à la fois l'activité de la oLH et de la oFSH (9A4-A7-D3 et 9A4-D4-B6), cet effet potentialisant étant de 150% pour les points 6,2 ng/ml, 12,5 ng/ml et 25 ng/ml d'hormone. Ces 3 anticorps sont tous d'isotype IgM.

Les séquences nucléotidiques de la région variable des chaînes lourdes et des chaînes légères des anticorps 9A4 A7 D3, 1A6 C4 G11 et 9A4 D4 B6 ont été déterminées comme suit. Les ARN totaux ont été extraits à partir de 10⁹ cellules d'hybridomes, en utilisant le réactif RNABle^{®} (EUROBIO, France), en suivant le protocole préconisé par le fabricant. Les ARN ont ensuite été isolés et un dosage spectrophotométrique à 260/280 nm a été effectué pour connaître la concentration en ARN des échantillons. Par technique de RT-PCR (« *reverse transcription-polymerase chain reaction* »), la séquence complémentaire d'ADN a été obtenue à partir de chaque brin d'ARN. Le milieu réactionnel était composé de 4 µl d'ARN à 2 µg/ml auquel il a été ajouté 20 µl d'Oligo dT à 100 ng/µl et 38,2 µl d'eau MilliQ. Après avoir chauffé l'échantillon pendant 5 minutes à 70°C, 20 µl de tampon 5X et 10 µl de dNTP ont été ajoutés. Ce volume a été complété avec 2 µl de reverse transcriptase et 3,2 µl de RNAse et le tube a été placé pendant 1h à 42°C. Le milieu réactionnel utilisé était composé de 2 µl de MgCl₂ à 25 mM, 8 µl des quatre dNTP à 2,5 mM, environ 1U de Taq polymerase^{®} et 5 µl de tampon réactionnel 10X. Les deux amorces «sens» et «anti-sens» (1,5 µl) ont ensuite été ajoutées ainsi que l'ADNc (3 µl) puis le volume final a été complété à 50 µl avec de l'eau MilliQ. Neuf couples d'amorces ont été utilisés pour amplifier les VL et deux amorces ont été utilisées pour les VH. Leurs séquences sont décrites par Peter et al. (The Journal of Biological Chemistry, 278, 36740-36747, 2003) et par Mousli M. et al. (FEBS Letters 442: 183-188, 1999).

Le nombre de cycles de PCR a été de 30, comprenant chacun 1 minute à 90°C, 1 minute à 47°C puis 3 minutes à 72°C.

Les produits PCR ont été analysés par électrophorèse sur gel d'agarose à 2% coloré au bromure d'éthidium, purifiés avec le kit « QIAquick PCR Purification » (QIAGEN, Pays-Bas) et séquencés. L'alignement multiple des séquences VH et VL obtenues a été réalisé à l'aide du logiciel Multalin (F. Corpet, Nucl. Acids Res., 16 (22), 10881-10890, 1988).

Les séquences d'acides aminés des boucles hypervariables CDR1, CDR2 et CDR3 des régions variables des anticorps 9A4 A7 D3, 1A6 C4 G11 et 9A4 D4 B6 ont été déduites à partir des séquences oligonucléotidiques des chaînes lourdes et des chaînes légères ci-dessus, à l'aide de la base IMGT/V-QUEST. L'alignement et la délimitation des régions framework et CDR des régions variables se sont faits selon le référencement de la base FR-IMGT et CDR-IMGT (IMGT/3Dstructure-DB ; Kaas Q et al., Nucleic Acid Research, 32: 208-210,2004).

### EXEMPLE 2 : MESURE DE L'EFFET POTENTIALISANT DES ACM IN VIVO, CHEZ LE RAT

Par culture *in vitro,* 10 mg de chaque anticorps monoclonal de l'Exemple 1 ont été produits. Après purification et concentration, ils ont été testés *in vivo.*

Le modèle du rat a été choisi du fait qu'il est la référence internationale utilisée par la pharmacopée, pour mesurer l'activité des hormones gonadotropes.

Les anticorps anti-oLH obtenus ne croisant pas avec la LH de rat, une hormone exogène humaine, la hCG, a été utilisée pour tester l'effet potentialisant de ces anticorps. La hCG est très bien reconnue par les anticorps anti-oLH et présente l'avantage d'avoir une activité LH stricte. De plus, elle est facilement disponible commercialement sous une forme très pure et peu onéreuse.

Deux dosages biologiques référentiels utilisés par la pharmacopée pour doser l'activité LH ont donc été utilisés : l'un chez le mâle (Scobey MJ et al., 2005, Reprod. Biol. Endocr. 3 : 61) et l'autre chez la femelle (Parlow A F, 1958, Fed. Proc. 17 : 402).

Le dosage chez la femelle fait l'objet de l'Exemple 3.

Chez le mâle, la bioactivité de la LH ou de la hCG est quantifiée par rapport à l'augmentation de la masse des vésicules séminales. Des ratons de 25 jours sont injectés avec l'hormone seule (1,5 UI) ou préalablement incubée avec l'anticorps, une fois par jour pendant 4 jours puis sacrifiés le 5^{ème} jour pour mesurer le poids des vésicules séminales. Celui-ci varie proportionnellement à l'activité de la LH, le développement des vésicules séminales étant très androgéno-dépendant.

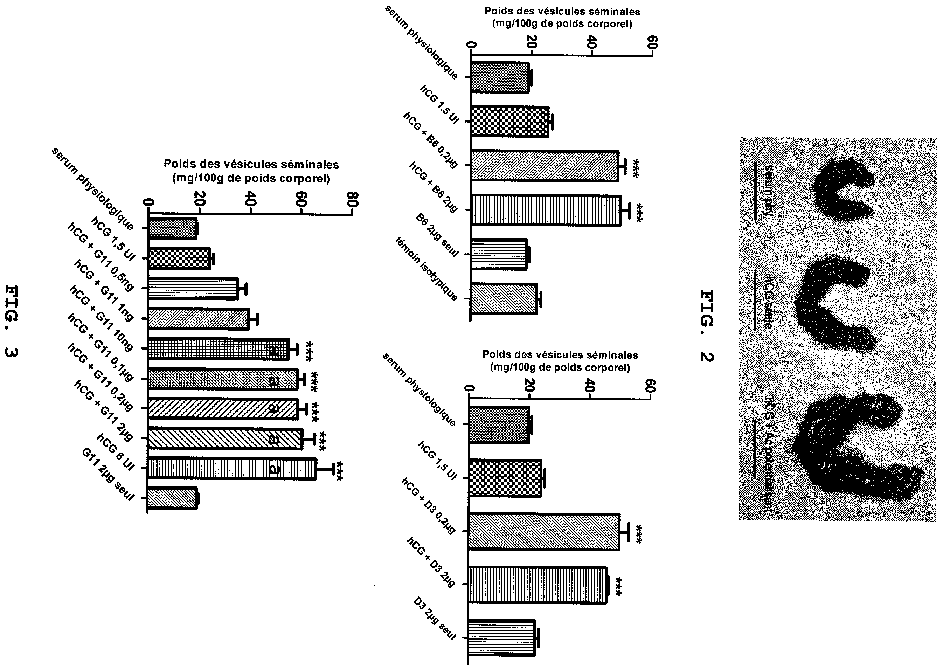

La Figure 2 illustre l'effet potentialisant exercé par l'anticorps potentialisant B6 sur la bioactivité de la hCG, en observant la taille des vésicules séminales d'un rat témoin traité avec du sérum physiologique, d'un rat traité avec 1,5 UI de hCG et d'un rat traité avec 1,5 UI d'hCG préincubée avec 2 µg d'anticorps B6.

La Figure 3 montre l'effet potentialisant des AcM B6, D3 et G11 sur l'augmentation du poids des vésicules séminales. Ces résultats ont été obtenus avec des lots de 8 rats et chaque expérience a été répétée deux fois. Chez les rats traités avec le complexe hCG/AcM, le poids des vésicules séminales est doublé par rapport au poids des vésicules des rats traités avec l'hormone seule. Une analyse statistique a été réalisée avec le logiciel GraphPad Prism (GraphPad PRISM Software; GraphPad, San Diego, CA) par analyse de variance à une variable et par le test de Bonferroni (« *Bonferroni's Multiple Comparison Test* »). Elle a montré que, pour les trois AcM (B6, D3, G11), le poids des vésicules séminales obtenu dans le lot traité avec hCG 1,5 UI + AcM à 0,2 µg ou 2 µg, par injection, est statistiquement très différent de celui du lot traité avec hCG seule (p<0,001). Les rats traités avec l'anticorps seul ou avec un anticorps témoin isotypique (IgM normal) présentent un poids de vésicules séminales égal à celui du rat témoin, indiquant que l'anticorps seul n'a pas d'effet.

L'effet potentialisant est obtenu avec des doses très faibles d'anticorps. Dès la dose de 0,5 ng d'AcM, on observe une augmentation du poids des vésicules séminales chez les rats traités avec le complexe hCG+AcM G11. Aux doses de 0,5 et 1 ng d'AcM G11, l'augmentation du poids montre une tendance potentialisante mais non significative. L'effet potentialisant du complexe devient très significatif (p<0,001) à partir de la dose hCG 1,5 UI + AcM 10 ng par injection. Il faut remarquer que les poids obtenus avec les doses hCG 1,5 UI + respectivement 10 ng - 0,1 µg - 0,2 µg ou 2 µg d'AcM G11 ne sont pas différents statistiquement entre eux. De même, ils ne sont pas différents du poids des vésicules séminales obtenus chez des rats traités avec 6 UI de hCG seule. Ceci veut dire que le complexe 10 ng d'AcM G11 + hCG 1,5 UI est capable d'induire le même niveau de stimulation que 6 UI de hCG seule : son effet potentialisant permet de multiplier par un facteur 4 l'effet de l'hormone seule. Cet effet est quasiment maximum dès 10 ng d'AcM, traduisant une très grande sensibilité et efficacité de cet AcM. Des résultats identiques ont été obtenus avec les AcM B6 et D3 (non montrés).

L'absence d'effets secondaires suite aux traitements avec les complexes a été vérifiée en pesant les testicules et les épididymes des animaux et en faisant une étude histologique des gonades. Celle-ci était parfaitement normale et aucune anomalie n'a été constatée dans les poids des testicules et des épididymes.

### EXEMPLE 3 : MESURE DE L'EFFET POTENTIALISANT DES ACM IN VIVO, CHEZ LA RATTE

Chez la ratte, la bioactivité de la LH est quantifiée par la baisse du taux d'acide ascorbique présent dans les ovaires, selon le dosage de Parlow (1958, précité). Après un pré-traitement avec hCG à J0 et avec eCG à J2 pour la lutéinisation des ovaires, les rattes sont traitées à J8, soit avec de la hCG seule soit avec de la hCG préalablement incubée avec un anticorps.

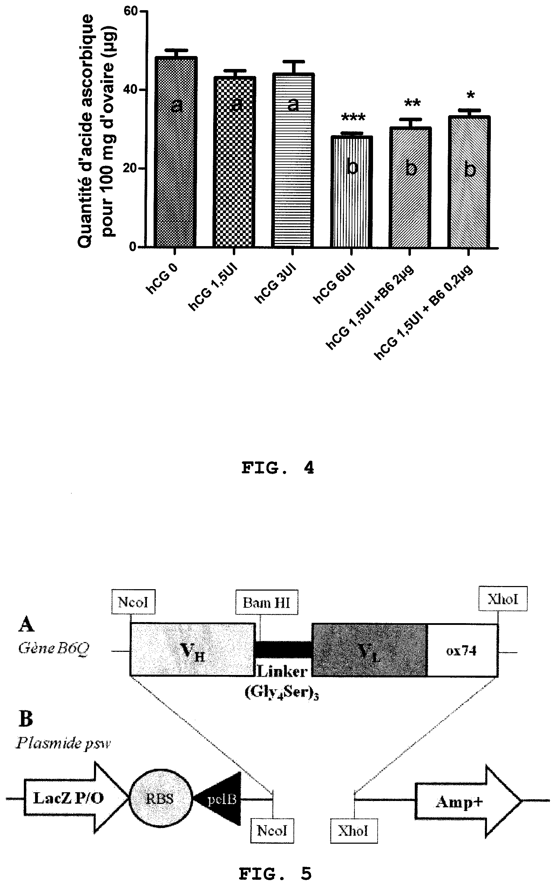

La Figure 4 montre l'effet potentialisant exercé par l'ACM B6 chez la ratte. Une analyse statistique a été réalisée avec le logiciel GraphPad Prism (GraphPad PRISM Software; GraphPad, San Diego, CA) par analyse de variance à une variable et par le test de Bonferroni (Bonferroni's Multiple Comparison Test). Une baisse significative du taux d'acide ascorbique est observée dans les ovaires des rattes traitées avec le complexe hCG 1,5UI + B6 2µg (*p*<*0,01*) ou 0,2 µg (*p*<*0,05*) et hCG 6UI seule (*p*<*0,001*)*.* Les résultats obtenus montrent que le complexe hCG 1,5UI + anticorps entraînent une diminution du taux d'acide ascorbique équivalente à une dose de 6UI de hCG injectée seule. L'effet du complexe est donc équivalent à celui d'une dose d'hCG 4 fois supérieur.

Le même type de résultat a été obtenu avec les deux autres ACM (résultats non montrés). Aucun ACM n'exerce d'effet lorsqu'il est injecté seul.

En conclusion, les résultats des exemples 2 et 3 démontrent qu'un effet potentialisant du complexe hormone/anticorps s'exerce très nettement *in vivo,* entraînant une réponse stéroïdogène amplifiée dans les organes cibles. Ils démontrent ainsi sans ambiguïté que le concept de potentialisation de l'activité d'une hormone gonadotrope est applicable *in vivo* chez le rat mâle comme chez la femelle.

### EXEMPLE 4 : CONSTRUCTION DES FRAGMENTS SCFV

Le gène de synthèse du scFv B6 a été élaboré à partir des séquences de l'anticorps B6 avec deux acides aminés modifiés : l'acide aminé 1 (QVQ au lieu de EVQ) du framework 1 de la chaîne lourde et l'acide aminé 3 (DIQ au lieu de DIV) du framework 1 de la chaîne légère. Ce gène de synthèse a été inséré dans un plasmide pcr4-TOPO et contient à ses 2 extrémités les sites de restriction NcoI et XhoI.

Le gène de synthèse est représenté schématiquement à la Figure 5A. Les codons ont été optimisés pour l'expression du scFv dans *E. coli.* La séquence du gène a été conçue pour pouvoir obtenir le domaine VH et le domaine VL reliés par un lieur peptidique. Ce dernier est constitué de résidus glycine et sérine pour conférer au lieur peptidique flexibilité et résistance aux protéases (Bird et al., Science, 242(4877):423-426, 1988). Lorsque ce lieur est d'une taille supérieure à 12 acides aminés, les domaines variables s'associent dans leur conformation originale pour former le site de liaison à l'antigène (Whitlow et al., Protein Eng., 7(8): 1017-1026, 1994). A l'extrémité 3' de la construction, on trouve une séquence codant pour un peptide de 14 résidus. Ce peptide drapeau MRC-OX74 fusionné au niveau du C-terminal de la protéine recombinante facilite sa détection (Cyster et al., European Journal of Immunology, 22(10): 2565-2572,1992).

Les deux sites de restriction, NcoI et Xhol, aux extrémités du gène de synthèse permettent son insertion dans le plasmide pSW1 (Figure 5B).

Le plasmide pSW1 a été utilisé pour construire et exprimer le gène codant le fragment d'anticorps recombinant. Le plasmide contient, sous le contrôle d'un promoteur inductible LacZ, la séquence signal pelB à laquelle est fusionnée, en phase de lecture, la séquence correspondant au gène de synthèse qui a été développé. La séquence signal pelB va permettre l'adressage du scFv dans le périplasme. Le plasmide contient également un site de fixation du ribosome (RBS) et un gène de résistance à l'ampicilline (Amp+).

Le gène de synthèse a été excisé de pCR4-TOPO en utilisant 0,5 µl des endonucléases PstI et XhoI (Promega, USA) pour 6 µl de plasmide pendant 1h30 à 37°C dans le tampon préconisé par le fournisseur. La même digestion a été effectuée sur le plasmide pSW1. Les produits obtenus ont ensuite été analysés par électrophorèse sur gel d'agarose en présence de bromure d'éthidium.

Les bandes d'intérêt ont été isolées et purifiées à partir du gel d'agarose et la présence de l'insert ou du vecteur digéré et leur concentration a été vérifiée avec le dépôt de 2jt1 de l'éluat par électrophorèse sur gel agarose 1%.

Le vecteur pSW1-scFvB6 est obtenu par ligation de l'insert B6 avec le plasmide pSW1. Pour cela, dans un volume réactionnel de 10 µl, l'insert (2 µg) est incubé avec 6 µg de plasmide coupé et déphosphorylé dans un tampon de ligation auquel sont ajoutés 1,5 µl d'eau et 0,5 µl de T4 DNA ligase (PROMEGA, USA). En parallèle, un contrôle est effectué sans insert. Le tout est placé à 15°C pendant 14h puis à 4°C durant 4h.

La souche *E. coli* HB2151 est utilisée pour la transformation bactérienne. Celle-ci est effectuée, de manière classique, à partir d'un produit de ligation ou à partir de plasmides déjà purifiés. La souche d'*E. coli* HB2151 est rendue compétente de manière chimique en utilisant du chlorure de calcium 1M. Le plasmide ou le produit de ligation (2 µl) sont mis en contact avec 200 µl de ces bactéries pendant 30 minutes à 4°C. Ensuite, les bactéries subissent un choc thermique pendant 90 secondes dans un bain à 42°C. Les bactéries sont immédiatement remises dans la glace pendant 2 minutes pour permettre l'incorporation du plasmide. Chaque transformation est diluée dans 800 µl de milieu de culture LB (Luria Bertani) et incubée pendant 45 minutes à 37°C sous agitation rotative (200 rpm) pour permettre l'expression du gène de résistance à l'ampicilline. Les bactéries ayant incorporé le plasmide referment leur paroi et expriment le gène de résistance à l'ampicilline. Elles sont ensuite étalées sur une boîte de Pétri contenant le milieu solide et incubées une nuit à 37°C. Les colonies formées sont alors analysées.

Après transformation, les clones positifs ont été sélectionnés sur milieu solide composé d'agar en présence d'ampicilline. Les bactéries sélectionnées sont mises en culture pour en extraire les plasmides recombinants. Pour vérifier la présence de l'insert, l'ADN plasmidique a été digéré par les mêmes enzymes de restriction que celles utilisées pour le clonage. La migration des produits de cette digestion sur gel d'agarose 1% a permis de mettre en évidence 2 clones (nommés 26 et 27) possédant l'insert. Ces derniers ont été séquencés afin de vérifier que le cadre de lecture de la séquence est en phase et que l'insert ne possède aucune mutation.

### Culture bactérienne

Les bactéries *Escherichia coli* positives ont été cultivées en aérobiose à 37°C, soit en milieu liquide sous agitation (200 rpm), soit en milieu solide. Les milieux de culture LB ont été stérilisés par autoclave, pour obtenir les milieux solides, 15 g d'agar par litre de LB ont été additionnés. L'antibiotique de sélection des bactéries recombinantes est l'ampicilline. Il a été ajouté à une concentration de 100 µg par ml. Les souches *E. coli* sont conservées à 4°C en milieu solide pour une durée maximale de 4 semaines. Pour une conservation à plus long terme, les souches bactériennes peuvent êtres conservées à -80°C dans du LB contenant 15% (v/v) de glycérol stérile. La densité bactérienne en milieu de culture liquide peut être estimée en mesurant l'absorption à 600 nm : 1 unité de DO à 600 nm = 8x10⁸ bactéries/ml.

### Induction bactérienne

L'induction bactérienne avec le milieu de culture LB a été effectuée par addition d'IPTG (isopropyl β-D-1-thiogalactopyranoside), lorsque la culture est en phase de croissance stationnaire avec une densité optique supérieure à 1,2. Une pré-culture a été lancée avec la colonie bactérienne d'intérêt la veille dans un milieu de culture LB contenant de l'ampicilline. Le lendemain, une culture de plus grand volume (500 ml) a été ensemencée en ajoutant la pré-culture au 1:1000. L'induction est effectuée avec l'ajout de 0,84 mM d'IPTG lorsque la culture atteint une DO supérieure à 0,6. La culture a été mise à incuber sous agitation (130 rpm) pendant 16 heures à 17°C.

Après 16 heures d'expression de la protéine recombinante, les protéines périplasmiques sont isolées par choc osmotique ménagé. La bonne production du scFv a été vérifiée sur gel SDS-PAGE puis par un Western Blot en conditions dénaturantes. Ce test est suffisamment sensible pour démontrer la bonne production et l'exportation de la protéine recombinante dans le périplasme des bactéries.

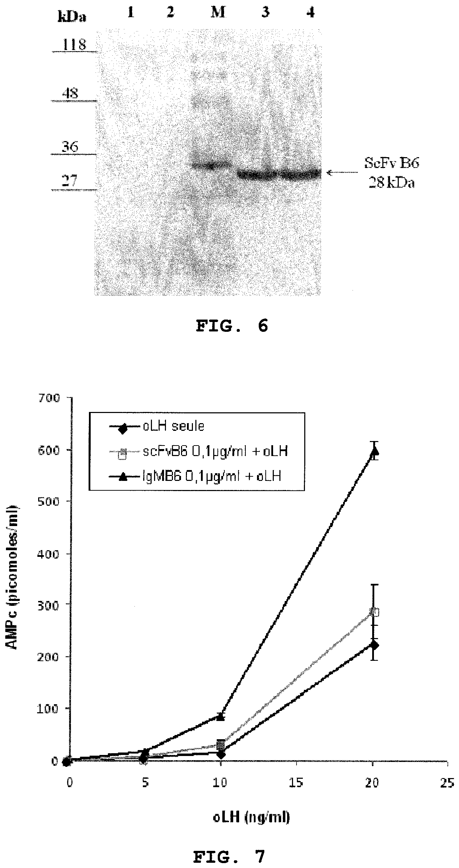

La Figure 6 montre les résultats de l'analyse par Western Blot de l'expression du scFv B6 par E. coli. Les pistes 1 et 2 correspondent à un système d'expression n'ayant pas produit le scFv B6 (plasmide pHEN dans la souche BL21 d'*E. coli*). Les pistes 3 et 4 correspondent respectivement aux clones 26 et 27 qui expriment le scFv B6. Piste M : marqueur de poids moléculaire en kDa. On observe la présence d'une bande unique d'une masse d'environ 28 kDa.

La protéine issue de cette bande à 28kDa est reconnue par l'anticorps anti-OX74 en immunoblotting et montre ainsi que la protéine produite correspond au scFv B6.

### Extraction du scFv

Toutes les étapes de l'extraction et de la dialyse ont été réalisées à 4°C. Pour extraire les protéines recombinantes du périplasme des bactéries, les cultures sont centrifugées à 5000 rpm pendant 20 minutes. Pour rompre la membrane externe, le culot bactérien a été repris dans un volume de TES (Tris-HCl 30 mM, EDTA ImM, sucrose 20 %, pH 8,5) correspondant au 1:50 du volume de la culture bactérienne. L'échantillon a été placé sur la glace durant 1/4 d'heure et vortexé à intervalles réguliers. Cette étape a été répétée une seconde fois en utilisant le même tampon de lyse dilué au 1/4 et représentant cette fois-ci 1:33,33 du volume de la culture. Après centrifugation à 10000 rpm pendant 30 minutes, le périplasme a été mis en dialyse toute la nuit sous agitation contre 5 litres de PBS (NaCl 0,14 M, KCl 13 mM, KH₂PO₄ 9 mM, Na₂HPO₄ 50 mM, pH 7,4).

### Purification du scFv B6

Le kit Carboxyl-Adembeads (Ademtech, France) a été utilisé pour purifier le scFv B6 de la préparation de protéines périplasmatiques en suivant le protocole fournit par le fournisseur. Le groupement carboxylique des billes magnétiques utilisées dans ce kit a été activé permettant ainsi de créer une liaison peptidique avec l'anticorps anti-OX74. Le périplasme a ensuite été incubé avec cette préparation pendant 3h à 37°C, le scFv B6 a été reconnu par l'anticorps fixé grâce à la présence du peptide drapeau OX74. L'élution des protéines retenues est faite avec une solution de glycine-HCl 0,1 M à pH 2,0. Des fractions de 1 ml ont été recueillies et le pH a été ramené immédiatement à une valeur de 7,5 par ajout de 50 µl de Tris 1 M. Les fractions éluées dont l'absorbance à 280 nm est supérieure à 0,1 ont été rassemblées puis dialysées contre du tampon PBS pendant une nuit et ont ensuite été concentrées sur concentrateurs Minicon® (MILLIPORE, Irlande).

La séquence peptidique du fragment scFv B6 est représentée dans la liste de séquences en annexe sous le numéro SEQ ID NO: 28. Cette séquence SEQ ID NO: 28 n'inclut pas le peptide drapeau MRC-OX74.

La séquence du gène codant le scFv B6 peut être modifiée par mutagénèse dirigée afin d'obtenir, dans la séquence peptidique, un résidu proline (P) à la place d'un résidu thréonine (T), en position 145 de la SEQ ID NO: 28. Cette mutation confère au scFv la propriété de pouvoir être reconnu par la protéine L et ainsi être purifié par chromatographie d'affinité sur protéine L.

### Analyse protéique

Dans cette étude, la technique de SDS-PAGE a été utilisée afin de caractériser la présence du scFv dans le périplasme des bactéries. Le choix de la concentration du gel d'acrylamide (12%) se fait en fonction de la taille de la protéine d'intérêt (Sambrook *et al.,* 1989, précité). Avant d'être déposé, l'échantillon a été dilué dans du tampon de charge (Tris-HCL 100 mM, pH 6,8, SDS 4%, β-mercaptoéthanol 1%, bleu de bromophénol 0,2%, glycérol 20%) et dénaturé à 95°C pendant 5 minutes. La migration des protéines s'est effectuée dans un tampon d'électrophorèse à 60 Volts au sein du gel de concentration puis à 150 Volts lorsque les protéines ont pénétré dans le gel de séparation. Le gel a ensuite été incubé dans une solution de bleu de Coomassie (bleu de Coomassie 0,25% (p/v), méthanol 50% (v/v), acide acétique 10% (v/v)) pendant au minimum 30 minutes sous agitation. L'excès de coloration a été éliminé avec des lavages successifs dans une solution de décoloration (éthanol 25% (v/v), acide acétique 7% (v/v)). Les bandes bleues présentes sur le gel correspondent aux protéines contenues dans l'échantillon. Les protéines qui ont été séparées par électrophorèse ont été transférées de manière passive pendant une nuit, sur une membrane de nitrocellulose en contact avec le gel. Cet ensemble est entouré de plusieurs épaisseurs de papier Whatman trempé dans le tampon de transfert (Tris 25 mM pH 8,3, glycine 129 mM, SDS 0,01% (p/v), méthanol 20% (v/v)).

A la fin du transfert, les sites libres de la membrane de nitrocellulose ont été saturés pendant une heure à température ambiante dans une solution de tampon de transfert avec 5% (p/v) de lait déshydraté. La membrane a ensuite été incubée pendant une heure avec du surnageant de milieu de culture contenant l'anticorps anti-OX74 spécifique du peptide ajouté en C-terminal du scFv. Après trois lavages dans le tampon de transfert, la membrane a été mise à incuber pendant une heure avec une solution qui cette fois-ci contient l'anticorps secondaire (anticorps anti-IgG de souris couplé à la phosphatase). Finalement la membrane a été lavée trois fois, la révélation a été effectuée en incubant pendant quelques secondes avec un substrat couplé à la phosphatase alcaline (BCIP/NBT, (bromochloroindolyl phosphate / NitroBlue Tetrazolium)) dilué au demi dans le tampon de révélation.

### Analyse de la spécificité du scFv vis-à-vis de la LH et quantification par ELISA

La spécificité du scFv vis-à-vis de la oLH a été mesurée par ELISA. Pour cela, les périplasmes des clones 26/27 et un périplasme contrôle sans molécules reconnaissant la oLH ont été testés.

Pour effectuer le test ELISA, 100 µl d'une solution de oLH à 1 µg/ml (dilué dans du tampon carbonate-bicarbonate 0,1 M, pH 9,6) sont déposés par puits d'une plaque (Nunc Immuno Plate) pendant 1h à 37°C puis une nuit à 4°C. Les sites résiduels sont bloqués par une solution saturante avec 100 µl d'une solution de PBS - Tween 0,1% - BSA 1%, à 37°C pendant 45 minutes. Les différentes dilutions de périplasmes sont déposées en duplicat à raison de 100 µl par puits, la plaque est mise à incuber à 37°C pendant 1h. Le dépôt de l'anticorps primaire du scFvB6 est effectué en ajoutant 100 µl d'une solution composée de surnageant de culture, contenant l'anticorps anti-OX74 dilué au tiers dans du tampon PBS-Tween 0,01% - BSA 0,01% après avoir effectué 5 lavages au PBS - Tween 0,1%. Cet anticorps est reconnu par un IgG de souris couplé à la peroxydase dilué au 2500ème dans le même tampon que l'anticorps primaire, 100 µl ont été ajoutés par puits. Après avoir de nouveau lavé 5 fois la plaque, elle est placée à 37°C pendant 1h. Ensuite, 100 µL de TMB (3,3',5,5'-tetramethylbenzidine, Kirkegaard & Perry Laboratories Inc., USA), un substrat chromogénique de la peroxydase, sont ajoutés dans chaque puits. Après 25 à 30 min d'incubation, la réaction est arrêtée par 100 µL d'acide sulfurique (H₂SO₄ 1 M). L'absorbance de la solution est mesurée à 450 nm par un spectrophotomètre lecteur de microplaques.

A l'inverse du contrôle, un signal colorimétrique positif a été obtenu avec la protéine recombinante : le scFv reconnaît bien la oLH.

La concentration du périplasme a été mesurée par un dosage ELISA quantitatif. 100µl d'un anticorps primaire standard anti-oLH (10 - 5 - 1 - 0,5 - 0,1 - 0,05-0,025 µg/ml) ont été déposés par puits sur lesquels de la oLH a été préalablement adsorbée à 1 µg/ml. Après incubation d'un anticorps secondaire couplé à la peroxydase, et révélation avec du TMB, la lecture de l'absorbance à 450 nm a permis d'obtenir une gamme étalon permettant d'estimer la concentration en scFv des différentes dilutions de périplasmes testés allant du demi au 1/8^{ème}.

La concentration du périplasme des clones 26/27 a ainsi été estimée à 34 µg/µl.

Afin d'augmenter cette concentration et pour éliminer les protéines bactériennes nous avons purifié le scFv à partir de l'anticorps anti-OX74 couplé aux billes Ademtech. Les deux fractions d'élution ont été réunies en un seul échantillon et après dialyse pendant une nuit à 4°C, leur concentration a été estimée par mesure de l'absorbance à 280 nm. Les mesures de concentration sont présentées dans le Tableau V ci-dessous. L'absorbance mesurée à 280 nm à partir de plusieurs dilutions a permis d'estimer la concentration en scFv de la solution à 0,84 mg/ml.

**Tableau V :**

| DO 1 | DO 1/10ème | DO 1/20ème | Moyenne des DO | Quantité estimée de protéine |
|---|---|---|---|---|
| 1,211 | 0,106 | 0,068 | 1,21 | 0,84 mg/ml |

### EXEMPLE 5 : Etude de l'effet potentialisant du scFv sur la bioactivité de la oLH in vitro

L'effet potentialisant du scFv a pu être caractérisé, sur la bioactivité de la oLH et d'une hormone homologue d'un point de vue activité, la hCG. Pour cela, des dosages biologiques ont été effectués *in vitro* sur cellules MLTC et *in vivo* chez le rat (Exemple 6). Les tests *in vitro* ont été réalisés sur des cellules MLTC (Mouse Leydig Tumor Cell) qui expriment de façon stable le récepteur LH, et qui, lorsqu'elles sont stimulées avec de la LH ou de la hCG sécrètent de l'AMPc et de la progestérone (P4).

La réponse mesurée est la sécrétion en AMPc après 3 heures de stimulation à 37°C avec une gamme croissante de LH seule ou préalablement incubée avec le périplasme ou le scFv purifié. La production d'AMPc témoigne du couplage du récepteur LH activé à la protéine G stimulatrice (Gs) et donc de la transduction du signal via la voie Gs / adénylate cyclase / PKA (protéine kinase AMPc dépendante) / AMPc. La réponse est exprimée en picomoles d'AMPc sécrétées par 100000 cellules. La comparaison de la réponse biologique obtenue en présence de l'hormone seule et celle obtenue avec l'hormone préalablement incubée avec un surnageant permet de mesurer si celui-ci exerce un effet potentialisant ou aucun effet.

Les cellules MLTC ont été cultivées dans du milieu RPMI 1640 (additionné de L-glutamine et d'Hepes 25mM) (Gibco, USA) que l'on a supplémenté de 10% de FBS (foetal bovine serum), de 0,1% de gentamicine et d'un mélange de pénicilline et de streptomycine selon la méthode décrite par Martinat et collaborateurs (Martinat et al., Reprod Nutr Dev., 45(1):101-8, 2005). A 50% de confluence, les cellules sont trypsinisées puis ensemencées dans des plaques 24 puits à raison de 100000 cellules par puits (300 µl) et placées à l'étuve toute une nuit. Le sevrage des cellules se déroule le lendemain pendant 2 heures à 37°C, 5% de CO₂, en remplaçant le milieu de chaque puits par 200 µl de milieu de sevrage. Ce dernier est identique au milieu de croissance mais il est dépourvu de FBS et contient 240 µM d'IBMX (isobutylméthylxanthine) qui est un inhibiteur des phosphodiestérases. Il empêche la dégradation de l'AMPc au cours de la stimulation entraînant son accumulation, ce qui permet de bien évaluer l'efficacité de l'agoniste. En parallèle, une gamme de oLH (0 - 5 - 10 et 20 ng/ml final) ainsi que différentes concentrations du scFv à tester (0,1 et 1 µg/ml) ont été préparées dans un volume de 110 µl et préincubées pendant 1h à 37°C. A la fin du sevrage, les cellules MLTC ont été stimulées avec 50 µl des différents mélanges (scFv complexé ou non à la LH) et placées pendant 2h à l'étuve. Chaque point de stimulation de la LH seule ou des différents complexes a été testé en duplicate, sur deux puits de culture. Les surnageants ont ensuite été récupérés dans des tubes en verre et l'AMPc sécrété suite à la stimulation a été mesuré en utilisant un kit ELISA selon les instructions du fournisseur (Biomedical Technologies, Inc., Stoughton, USA).

En parallèle, l'IgM B6 purifié a lui aussi été testé pour voir si l'effet potentialisant que nous observons avec le scFv est plus ou moins important que celui obtenu avec l'anticorps B6 entier.

La Figure 7 illustre les résultats obtenus, lors d'une stimulation avec une gamme de oLH préincubée avec le scFv ou l'IgM, purifiés et testés à la concentration de 0,1 µg/ml. L'effet potentialisant du scFv purifié est faible et n'est pas significatif contrairement à celui de l'IgM B6 (*p*<*0,01*)*.* On remarque que le scFv seul, tout comme l'IgM seul, n'ont aucun effet sur la réponse cellulaire lorsqu'ils sont incubés sans oLH (point zéro de la courbe).

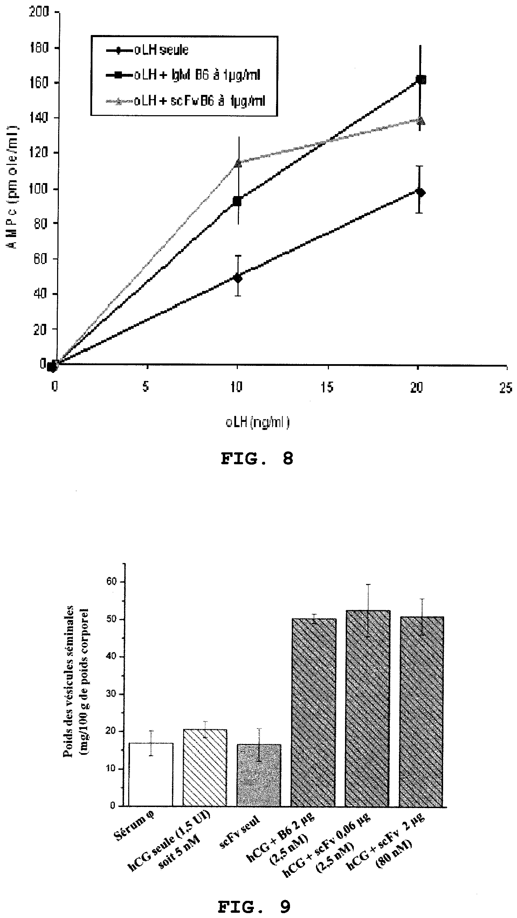

La Figure 8 illustre les résultats obtenus, lors d'une stimulation avec une gamme de oLH préincubée avec le périplasme ou l'IgM à la concentration de 1 µg/ml. On observe qu'à cette concentration, l'effet potentialisant du scFv est très proche de celui de l'IgM et est significatif (*p*<*0,05*)*.* Le périplasme seul n'a aucun effet sur la réponse (point zéro de la courbe).

### EXEMPLE 6: ETUDE DE L'EFFET POTENTIALISANT DU SCFV SUR LA BIOACTIVITÉ DE LA OLH IN VIVO, CHEZ LE RAT

L'objectif a été de vérifier que les effets observés *in vitro* sur la modulation de l'activité biologique de la oLH par le scFv peuvent aussi être observés *in vivo* sur la bioactivité de la hCG, analogue de la LH. Pour évaluer l'effet potentialisant du scFv B6, un dosage biologique référentiel utilisé par la pharmacopée pour doser l'activité biologique de la LH ou de la hCG chez le rat mâle est mis en oeuvre (Scobey *et al.,* 2005, précité). Dans ce dosage biologique, la bioactivité de la LH ou de la hCG est quantifiée par rapport à l'augmentation du poids des vésicules séminales dont le développement est très androgéno-dépendant. En raison du coût très élevé de la LH ovine, ces dosages biologiques ont été réalisés avec de la hCG, facilement disponible, sous une forme très pure et peu onéreuse. En effet, la hCG a une activité LH stricte et est très bien reconnue par l'anticorps potentialisant B6. Elle est considérée comme un analogue de la LH.

Le protocole a été réalisé avec des ratons de 25 jours qui ont été injectés avec l'hormone seule ou préalablement incubée avec le scFv ou l'anticorps B6, une fois par jour pendant 4 jours, puis sacrifiés le 5ème jour pour mesurer le poids des vésicules séminales. Celui-ci varie proportionnellement à l'activité de la hCG. Chaque condition a été testée sur un lot de 4 rats et a été répétée sur deux expériences indépendantes.

Les échantillons de scFv et de l'IgM B6 ont été préparés dans du sérum physiologique à plusieurs concentrations. Lors de précédentes expériences, le complexe hCG/IgM B6 a montré un effet maximal lorsque l'IgM est injecté à la concentration de 2,5 nM, soit 2 µg. Le poids d'un IgM pentamère est de 750 kDa tandis que celui du scFv est estimé à 25 kDa. Pour pouvoir comparer l'effet potentialisant de l'IgM et du scFv, ce dernier a été testé à une concentration également de 2,5 nM, soit 0,06 µg, afin de rester dans des conditions équimolaires. Le scFv a également été testé à la même quantité que l'IgM, soit 2 µg par injection.

Ces échantillons ont été pré-incubés ou non avec 1,5 UI de hCG à 37°C pendant une heure. Chaque rat a reçu 100 µl du mélange par injection. Le cinquième jour, les rats ont été pesés puis sacrifiés. Leurs vésicules séminales ont été prélevées et pesées. Le poids des vésicules séminales est exprimé en mg/100 grammes de poids corporel afin de pouvoir comparer les résultats obtenus avec les différents lots expérimentaux.

La Figure 9 illustre l'effet des complexes scFv/hCG et IgM/hCG sur le poids des vésicules séminales (n=1). Lorsque le scFv à 2 µg est injecté seul, il ne produit aucun effet : le poids moyen des vésicules séminales est du même niveau que celui du lot témoin qui a reçu une injection de sérum physiologique. Lorsque le complexe scFv/hCG est testé à la concentration de 2 µg et 0,06 µg, il entraîne une forte augmentation (250 %) du poids des vésicules séminales par rapport à l'injection de l'hormone seule. L'effet potentialisant du scFv à 2 µg atteint un niveau comparable à celui de l'IgM B6 injecté à la même quantité. En revanche, lorsque le scFv (0,06 µg) est testé en conditions équimolaires, son effet potentialisant est supérieur à celui de l'anticorps entier.

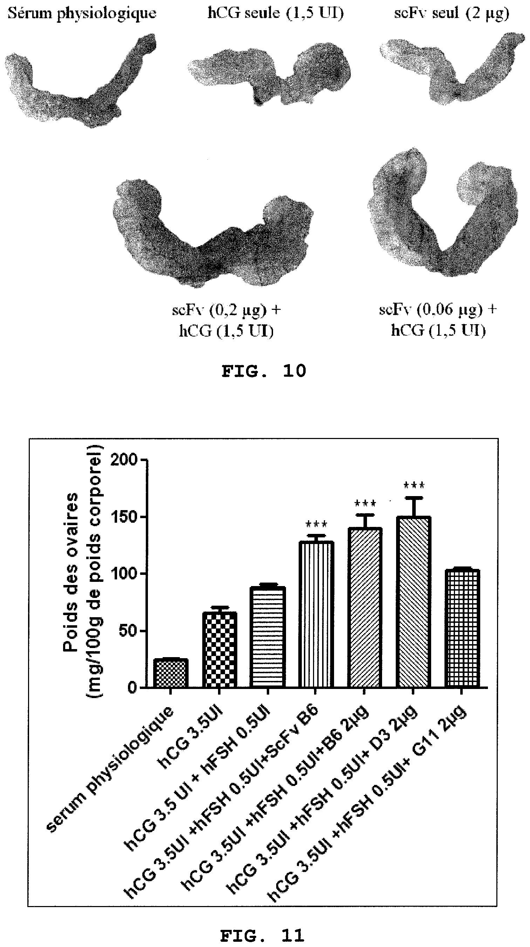

La Figure 10 illustre l'effet du complexe scFv/hCG sur la taille des vésicules séminales. Lorsque le scFv (0,2 µg et 0,06 µg) est injecté sous forme de complexe avec la hCG, il augmente la taille des vésicules séminales.

### EXEMPLE 7 : EFFET POTENTIALISANT DES ACM B6 ET D3 AINSI QUE DU SCFV B6 SUR L'ACTIVITE DE LA OFSH ET DE LA HFSH CHEZ LA RATTE

Afin de vérifier que l'effet potentialisant de la FSH des anticorps monoclonaux B6 et D3, observé *in vitro* sur cellules LTK (Exemple 1), était bien corrélé à un effet potentialisant de la FSH observé *in vivo,* leur effet potentialisant a été testé sur la bioactivité de la FSH ovine et humaine, chez la ratte.

Le protocole utilisé pour mesurer la bioactivité FSH est celui du dosage biologique décrit par Steelman et Pohley (Steelman SL, Pohley FM. Endocrinology, 53:604-616, 1953), utilisé comme protocole référence par la pharmacopée.

Des rattes immatures de 21 jours reçoivent pendant trois jours consécutifs 2 injections matin et soir de 100 µl d'un mélange de hCG et FSH. Les injections sont réalisées par voie sous-cutanée et comportent une quantité constante de hCG (3,5 UI) additionnée d'une quantité variable de FSH allant de 0,5 à 1,5 UI de FSH humaine (Gonal F, Merck-Serono) ou de 0,5 à 2 µg de FSH ovine. Afin de quantifier l'effet potentialisant de l'AcM testé, d'autres rattes sont traitées avec le même mélange ajouté de 2 µg d'anticorps purifié, ce mélange ayant été préalablement incubé 20 mn à 37°C.

Le quatrième jour, les rattes sont euthanasiées, pesées, et leurs ovaires sont disséqués puis pesés. Les résultats sont exprimés en milligramme d'ovaire/100 grammes de poids corporel. L'augmentation de poids des ovaires est proportionnelle à la quantité de FSH injectée ce qui permet de quantifier la bioactivité de la FSH injectée ainsi que l'effet potentialisant de l'AcM sur celle-ci.

Leur effet a été évalué sur la FSH humaine (Gonal F, hormone recombinante, Merck-Serono) utilisée pour la stimulation de l'ovulation chez la femme dans le cadre des traitements de procréation médicalement assistée (PMA). Cette hormone a été choisie en raison de sa grande pureté et de sa disponibilité. En effet, il a été montré précédemment que les AcM B6 et D3 exercent un effet potentialisant sur la oFSH, *in vitro* sur cellules LTK (Exemple 1). A l'inverse, l'AcM G11 n'a pas d'effet potentialisant sur la oFSH, *in vitro* sur ces mêmes cellules.

Dans cet exemple, les AcM B6 et D3 ont été testés à l'état d'anticorps entiers, ainsi qu'à l'état de scFv pour B6 (scFv B6).

L'anticorps G11 entier a également été étudié, en tant que contrôle de l'effet potentialisant LH strict.

Comme l'illustre la Figure 11, un effet potentialisant très significatif (*p<0,001*) a été obtenu avec B6, D3 et scFv B6 comparativement à l'effet du mélange hCG+hFSH injecté sans préincubation avec l'AcM. Ces résultats sont le cumul de deux expériences indépendantes et ont porté sur 8 animaux pour chaque lot. L'analyse statistique a été réalisée avec le logiciel GraphPad Prism (GraphPad PRISM Software; GraphPad, San Diego, CA) par analyse de variance à une variable et par le test de Bonferroni (*Bonferroni's Multiple Comparison Test*)*.*

A l'inverse, G11 ne donne pas d'effet potentialisant significatif lorsqu'il est injecté avec le mélange hCG+hFSH, ce qui corrèle bien avec son effet potentialisant LH strict.

Dans les expériences ci-dessus, les rattes ayant été traitées par un mélange de hCG et FSH, des expériences « contrôles » ont été menées afin de départager et de distinguer l'effet potentialisant exercé par ces AcM d'une part sur l'activité de la hCG et d'autre part sur l'activité de la FSH. En effet, deux expériences contrôles ont été réalisées, consistant à injecter :
(1) la FSH seule ou préincubée avec l'AcM D3, ou
(2) la hCG seule ou préincubée avec l'AcM D3.

L'AcM D3 a été choisi pour réaliser ces expériences car il donne l'effet potentialisant de la FSH le plus important. La FSH utilisée est la hFSH Gonal F (Serono, Merck).

### Expérience « contrôle » (1) :

L'objectif est de mesurer l'effet potentialisant des AcM sur la FSH seule. Les rattes ont donc été traitées avec de la FSH seule ou préincubée avec l'AcM.

Les rattes ont été traitées avec (a) FSH à 0,5 UI, seule ou préincubée avec D3 (2 µg), (b) FSH à 1 UI, seule ou préincubée avec D3 (2 µg), ou (c) FSH à 1,5 UI seule.

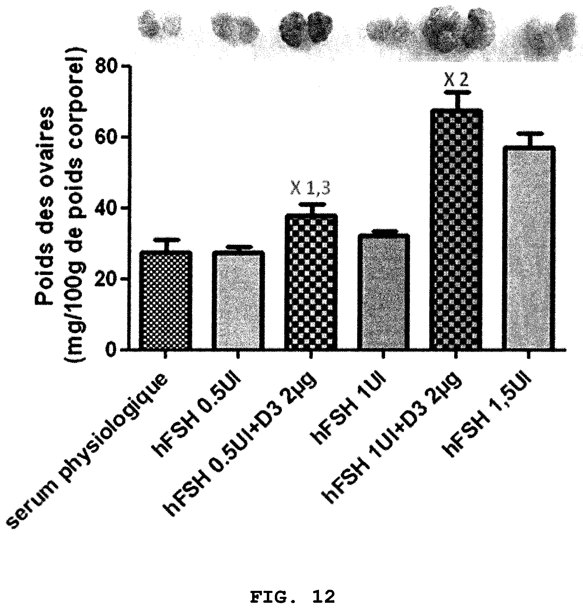

Les résultats sont illustrés par la Figure 12, dans un diagramme accompagné des photographies d'ovaire correspondantes. Le poids moyen des ovaires du lot traité avec FSH + D3 est significativement supérieur à celui du lot traité avec l'hormone seule, particulièrement à la dose de 1 UI FSH où on enregistre un doublement du poids des ovaires avec le complexe. Il est remarquable de souligner que la stimulation ovarienne observée avec le lot FSH 1 UI + D3 est supérieure à celle obtenue avec l'injection de 1,5 UI de FSH seule.

La comparaison du poids moyen des ovaires chez les rattes traitées avec l'hormone seule ou avec le complexe FSH/D3 met en évidence un effet potentialisant clair de l'AcM, particulièrement avec la dose 1 UI de FSH. Dans ce dernier cas, le poids moyen des ovaires est supérieur à celui obtenu chez les rattes traitées avec 1,5 UI FSH (n=5 rattes par lot).

### Expérience « contrôle » (2) :

L'objectif est de mesurer l'effet potentialisant des AcM sur la hCG seule. Pour cela, les rattes ont été traitées avec de la hCG seule ou pré-incubée avec l'AcM selon le protocole d'injections du dosage de Steelman et Pooley.

Les différents lots traités sont les suivants :
- hCG à 3,5 UI seule ou préincubée avec D3 (2 µg)
- hFSH à 0,5 UI seule
- hCG 3,5 UI + hFSH 0,5 UI
- hCG 3,5 UI + hFSH 0,5 UI + D3 2 µg

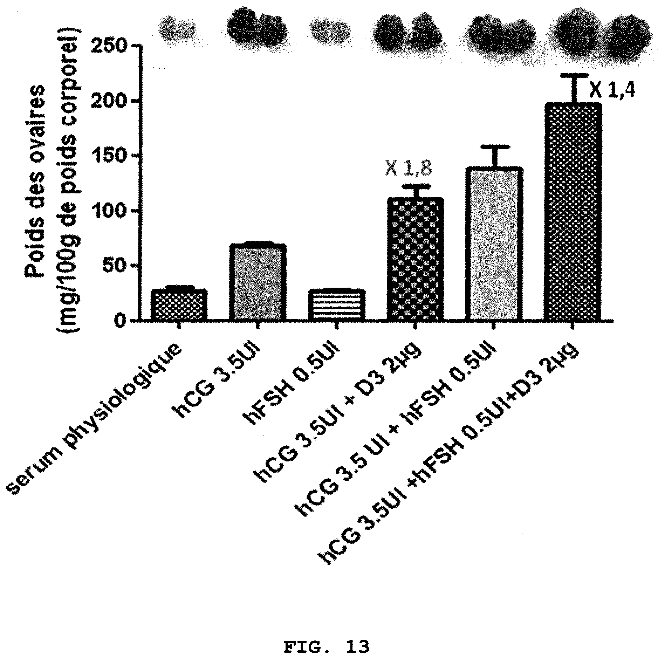

Les résultats sont illustrés par la Figure 13. Ils montrent que le poids moyen des ovaires du lot traité par hCG+D3 est supérieur au poids moyen des ovaires observé avec le lot traité par hCG seule. Il y a donc une potentialisation de hCG par l'AcM qui s'ajoute à celle exercée sur FSH.

Il est à noter également que l'effet observé avec le mélange AcM+hCG+hFSH entraîne une augmentation du poids des ovaires supérieure à l'addition des deux effets séparés : il y a donc un effet coopératif dans l'association des deux traitements (hCG et FSH).

En conclusion, l'effet observé avec le mélange AcM+hCG+hFSH est bien dû à un effet potentialisant de l'AcM sur la FSH, indépendamment de son effet potentialisant sur hCG. Ces expériences « contrôles » renforcent aussi la démonstration de l'effet potentialisant des AcM D3 et B6 sur la bioactivité de la FSH.

Ces résultats démontrent que les deux AcM, D3 et B6, exercent un double effet potentialisant, sur l'activité de la LH et sur celle de la FSH.

### EXEMPLE 8 : EFFET POTENTIALISANT DE L'ACM G11 IN VIVO, CHEZ LA BREBIS ILE DE FRANCE

Cette étude a été réalisée sur des brebis Ile de France, pubères, toutes du même âge. L'objectif a été d'évaluer, *in vivo* chez la brebis, l'effet potentialisant de l'AcM G11 sur l'activité d'une LH porcine (pLH) injectée pour induire l'ovulation. La pLH, extraite d'hypophyses de porcs, est utilisée dans certains traitements pour induire l'ovulation chez la brebis. Pour cela, on injecte 3 mg de pLH, par voie intraveineuse, 36 heures après le retrait de l'éponge.

Deux protocoles (A et B) ont été mis en place. Leur principe visait à évaluer l'effet potentialisant de G11 sur l'activité de la LH en datant d'une part le moment de l'ovulation et, d'autre part, la mise en place du corps jaune fonctionnel traduite par une augmentation de la sécrétion de progestérone pendant la phase lutéale. Pour cela, les paramètres physiologiques utilisés ont été les suivants :
- nombre et datation des ovulations déduits par observation endoscopique des corps jaunes réalisée par laparoscopie, sous anesthésie,
- mise en place et suivi de la sécrétion de progestérone par dosages plasmatiques quotidiens au cours de la phase lutéale.

Les tests ont été réalisés sur les mêmes brebis Ile de France, pubères, toutes du même âge (de 1 à 3 ans). Ces femelles ont toutes été synchronisées préalablement aux protocoles, par la pose d'une éponge vaginale imprégnée d'un progestagène (45 mg d'acétate de fluorogestone (FGA) - Intervet - France) pendant 14 jours.
Protocole A : l'effet potentialisant de G11 a été évalué en injectant le complexe pLH+AcM, préalablement incubé 30 minutes à 37°C :
   * pose d'éponges pendant 14 jours
   * injection du complexe pLH+AcM, par voie intramusculaire, 36 heures après le retrait de l'éponge
   * endoscopie entre 7 et 11 jours après retrait de l'éponge
   * prises de sang quotidiennes du premier jour au 21^{ème} jour après retrait de l'éponge, pour dosage de la progestérone dans le plasma
Protocole B : l'effet potentialisant de G11 a été évalué en injectant séquentiellement (1) la pLH, puis (2) 48 heures après, l'AcM :
   * pose d'éponges pendant 14 jours
   * injection de la pLH seule (3 mg) par voie intraveineuse, 36 heures après le retrait de l'éponge
   * injection de l'AcM seul (2 mg), par voie intramusculaire, 72 heures après le retrait de l'éponge, soit 48 heures après la pLH
   * endoscopie 11 jours après retrait de l'éponge
   * prises de sang quotidiennes du premier jour au 21^{ème} jour après retrait de l'éponge, pour dosage de la progestérone dans le plasma

### Résultats du protocole A :

Deux lots de 10 brebis ont reçu, par voie intramusculaire, 36 heures après retrait de l'éponge :
   - soit 3mg de pLH (lot pLH seule)
   - soit un mélange de 3mg de pLH + 2mg d'ACM G11 (lot pLH+ACM G11) incubé 30 minutes à 37°C préalablement à l'injection

### α- Analyses endoscopiques

Une endoscopie a été réalisée sur chaque brebis afin de contrôler s'il y a eu ovulation et afin de dater le ou les corps jaune(s) selon la méthode décrite par Cognié J. et collaborateurs (Revue Med.Vet. 2007, 158, 8-9, 447-451).

Pour chaque brebis, les résultats des endoscopies ont permis, d'une part, de préciser le nombre des ovulations (par comptage des corps jaunes) et, d'autre part, d'évaluer le moment de l'ovulation par rapport au retrait de l'éponge (par datation des corps jaunes).

Dans le lot pLH seule, toutes les brebis ont ovulé et ont eu une phase lutéale normale.

Dans le lot pLH+AcM, toutes les brebis ont ovulé. Une seule a eu une phase lutéale courte (cycle court) avec un corps jaune régressé précocement. Les 9 autres ont eu une phase lutéale normale. Cette différence n'est pas significative entre les deux lots.

Les résultats des ovulations sont résumés dans le Tableau VI ci-dessous.

**Tableau VI :**

| | Lot pLH seule | Lot pLH + AcM G11 |
|---|---|---|
| Nombre de brebis sans ovulation | 0 | 0 |
| Nombre de brebis ayant un CJ régressé et présentant une phase lutéale courte | 0 | 1 |
| Nombre de brebis ayant ovulé et présentant une phase lutéale normale | 10 | 9 |

Comme illustré par le Tableau VII ci-dessous, le moment moyen de l'ovulation s'est situé 2,5 jours après retrait de l'éponge dans le lot pLH+AcM G11 contre 3,5 jours dans le lot pLH seule. L'analyse statistique par test T indique que cette différence est significative à p<0,1 (GraphPad PRISM Software; GraphPad, San Diego, CA). Aucune différence significative n'a été observée dans le nombre d'ovulations entre les deux lots.

**Tableau VII :**

| | Lot pLH seule | Lot pLH + AcM G11 |
|---|---|---|
| Nombre moyen de corps jaunes par brebis | 2 ± 0,7 | 1,7 ± 1,06 |
| Moment moyen de l'ovulation (en jours après retrait de l'éponge) | 3,5 ± 1,5 | 2,5 ± 0,81 |

Le traitement par l'AcM complexé à la pLH induit donc une ovulation plus précoce présentant un décalage d'un jour par rapport au traitement par la pLH seule. Cette mise en place précoce traduit un effet potentialisant de l'AcM G11 sur l'activité LH.

### b- Mesure de la sécrétion de progestérone (P4) au cours du cycle sexuel

Des prises de sang journalières ont été effectuées à partir du jour de retrait des éponges (J0) et jusqu'au 21^{ème} jour. La progestérone a été dosée par méthode ELISA selon le protocole décrit par Canepa S. et collaborateurs (Cahiers Techniques INRA, 2008, 64, 19-30).

Seules les brebis ayant ovulé normalement ont été considérées, celle ayant eu une phase lutéale courte (cycle court) a été exclue.

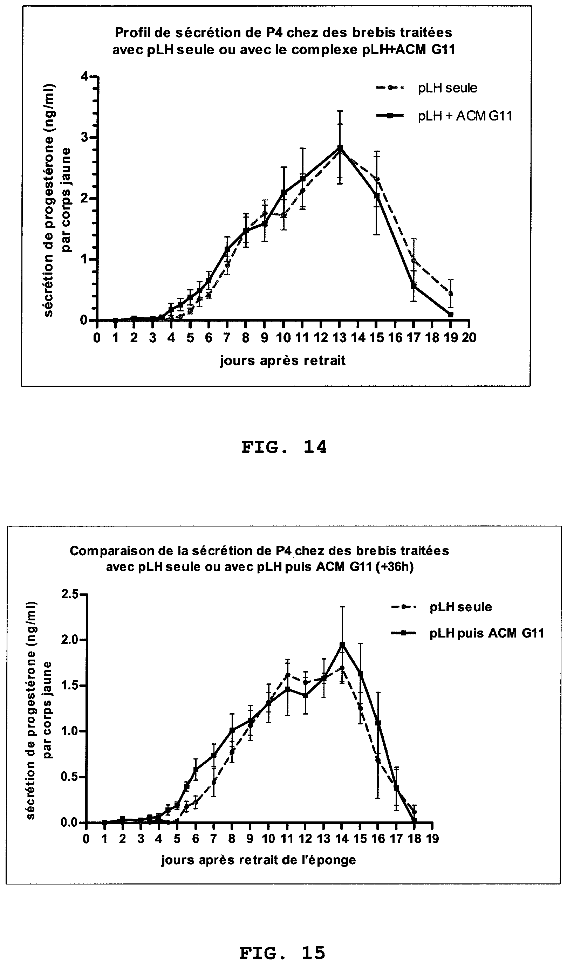

Pour chaque lot, les valeurs de concentration en progestérone, mesurées à chaque date du cycle, ont été moyennées. Afin de pouvoir moyenner les concentrations de P4 des femelles d'un même lot, la valeur basale de P4 mesurée à J1 après retrait de l'éponge a été arbitrairement considérée comme le niveau zéro de chaque femelle. De même, les résultats de P4 ont été exprimés pour un corps jaune : dans les cas où deux corps jaunes ont été observés chez une brebis, la valeur de P4 a été divisée par 2 pour chaque mesure. Les courbes moyennes obtenues pour chaque lot sont illustrées sur la Figure 14.

Pour comparer les deux courbes entières, l'analyse statistique a été faite par analyse de variances à deux variables (*two-way* ANOVA) à l'aide du logiciel GraphPad Prism (GraphPad PRISM Software; GraphPad, San Diego, CA). Elle montre que les deux courbes ne sont pas significativement différentes (*p*>*0,1*) ce qui indique que le complexe pLH+AcM G11 injecté par voie intramusculaire n'induit pas d'effet significatif avec cet effectif de 2X10 brebis. Cependant, une tendance, en faveur d'une précocité de 12 à 24 heures dans la mise en place de la sécrétion de P4, est observée signifiant que, chez les brebis du lot pLH+AcM G11, le corps jaune devient fonctionnel environ 24h plus tôt que chez les brebis du lot pLH seule (4 jours versus 5 jours respectivement). Ce décalage s'observe jusqu'au huitième jour après le retrait de l'éponge.

Dans le lot pLH+AcM G11, la tendance à une précocité de la mise en place d'un corps jaune fonctionnel est corrélée à une précocité significative du moment de l'ovulation observée par endoscopie. En effet, on obtient dans les deux cas une précocité de 24 heures, en faveur du lot pLH+AcM G11, pour les deux évènements physiologiques.

L'ensemble de ces résultats indique que le complexe pLH+AcM G11 est capable de potentialiser l'activité de la LH, *in vivo,* chez la brebis. Cette potentialisation est traduite par une ovulation statistiquement plus précoce et une tendance à induire plus précocement une réponse stéroïdogène des cellules lutéales stimulées chez les brebis traitées par le complexe pLH+AcM G11 comparativement aux brebis traitées avec l'hormone seule.

### Résultats du protocole B :

L'objectif du protocole B a été d'évaluer si l'AcM injecté séparément de l'hormone et de façon décalée dans le temps était aussi capable d'exercer un effet potentialisant sur la LH circulante, in vivo, chez la brebis.

Pour cela, deux lots de 8 brebis ont été traités, l'un avec 3 mg de pLH seule, injectés 36 heures après retrait de l'éponge par voie intraveineuse (IV), et l'autre avec 3 mg de pLH, 36 heures après retrait de l'éponge (voie IV), puis avec 2 mg d'AcM G11 48h plus tard, par voie intramusculaire (IM).

### a- Analyses endoscopique

Une endoscopie a été réalisée sur chaque brebis afin de contrôler s'il y a eu ovulation et de dater le ou les corps jaune(s) selon la méthode décrite par Cognié J. et collaborateurs (2007, précité).

Pour chaque brebis, les résultats des endoscopies ont permis, d'une part, de préciser le nombre des ovulations, par comptage des corps jaunes (CJ), et, d'autre part, d'évaluer le moment de l'ovulation par rapport au retrait de l'éponge, par datation des corps jaunes.

Cinq femelles sur huit ont ovulé normalement dans le lot pLH et six sur huit dans le lot pLH/AcM. Deux brebis ont ovulé mais ont eu une phase lutéale courte.

Les résultats des ovulations sont résumés dans le Tableau VIII ci-dessous.

**Tableau VIII :**

| | Lot pLH | Lot pLH/AcM |
|---|---|---|
| Nombre de brebis sans ovulation | 1 | 2 |
| Nombre de brebis ayant un CJ régressé et présentant une phase lutéale courte | 2 | 0 |
| Nombre de brebis ayant ovulé et présentant une phase lutéale normale | 5 | 6 |

Comme illustré dans le Tableau IX ci-dessous, le moment moyen de l'ovulation s'est situé 2,83 jours après retrait de l'éponge dans le lot pLH/AcM G11 contre 3,71 jours dans le lot pLH seule. L'analyse statistique par test T indique que cette différence est significative à p<0,1 (GraphPad PRISM Software; GraphPad, San Diego, CA). Aucune différence n'a été observée dans le nombre d'ovulations.

**Tableau IX :**

| | Lot pLH | Lot pLH/AcM |
|---|---|---|
| Nombre moyen de corps jaunes par brebis | 1,14 ± 0,37 | 1,16 ± 0,4 |
| Moment moyen de l'ovulation (en jours après retrait de l'éponge) | 3,71 ± 1,11 | 2,83 ± 1,16 |

### b- Mesure de la sécrétion de progestérone (P4) au cours du cycle sexuel

Des prises de sang journalières ont été effectuées à partir du jour de retrait des éponges (J0) et jusqu'au 21^{ème} jour. La progestérone a été dosée par méthode ELISA selon le protocole décrit par Canepa S. et collaborateurs (2008, précité).

Seules les brebis ayant ovulé normalement ont été considérées, celles ayant eu une phase lutéale courte (cycle court) ont été exclues.

Pour chaque lot, les valeurs de concentration en progestérone, mesurées à chaque date du cycle, ont été moyennées. Afin de pouvoir moyenner les concentrations de P4 des femelles d'un même lot, la valeur basale de P4 mesurée à J1 après retrait de l'éponge a été arbitrairement considérée comme le niveau zéro de chaque femelle. Par ailleurs, les résultats de P4 ont été exprimés pour un corps jaune : dans les cas où deux corps jaunes ont été observés chez une brebis, la valeur de P4 a été divisée par 2 pour chaque mesure. Les courbes moyennes obtenues pour chaque lot sont illustrées sur la Figure 15.

La compilation des résultats et leur analyse statistique ont été réalisées avec le logiciel GraphPad Prism (GraphPad PRISM Software; GraphPad, San Diego, CA). Pour comparer les deux courbes entières, l'analyse statistique a été faite par analyse de variances à deux variables (*two-way* ANOVA). Elle montre que les deux courbes sont significativement différentes (p<0,05) ce qui indique que l'AcM G11, injecté seul, exerce un effet potentialisant, significatif à p<0,05, sur l'activité de la LH.

Cet effet potentialisant se manifeste par une précocité de 24 heures dans la mise en place de la sécrétion de P4 (4,5 jours après retrait en moyenne) par rapport au lot pLH seule où la sécrétion se met en place 5,5 jours après retrait de l'éponge. Ces résultats signifient que, chez les brebis du lot pLH/AcM, le corps jaune devient fonctionnel 24h plus tôt que chez les brebis du lot pLH seule. Ce décalage s'observe de façon claire jusqu'au neuvième jour après le retrait de l'éponge.

Dans le lot pLH/AcM G11, la précocité de la mise en place d'un corps jaune fonctionnel est corrélée à une précocité du moment de l'ovulation observée par endoscopie. En effet, on obtient dans les deux cas un décalage de 24 heures à peu près entre les deux lots, avec à chaque fois une précocité des deux évènements physiologiques dans le lot traité avec l'AcM.

L'ensemble de ces résultats indique que l'AcM injecté seul, par voie intramusculaire, est capable de se lier à la LH ovine présente dans la circulation sanguine et de potentialiser son activité. Cette potentialisation est traduite par une induction plus rapide de la réponse stéroïdogène des cellules lutéales stimulées par le complexe LH plasmatique/AcM.

On remarque en outre que, la demi-vie de la pLH étant très courte (20 mn chez la brebis), cette hormone était totalement éliminée au moment de l'injection de l'AcM, décalée de 48 heures par rapport à celle de la pLH. Ceci signifie que les résultats obtenus sont dus à l'effet potentialisant de l'AcM G11 sur la LH endogène de l'animal. Ils démontrent donc que, chez un gros animal, ici la brebis, l'AcM, injecté seul, peut se complexer à la LH endogène et induire une potentialisation de son effet, *in vivo.*

### EXEMPLE 9 : CARACTERISATION DE LA SPECIFICITE DES ACM B6, D3 ET G11

La spécificité des AcM a été étudiée par technique ELISA. Chaque hormone évaluée a été adsorbée, 18h à 4°C, sur les puits d'une plaque ELISA à la concentration de 2 µg/ml dans du tampon carbonate de sodium 0,1 M à raison de 100 µl par puits.

Après cinq lavages (avec du PBS-Tween 0,1%) et une étape de surcoating (100 µl de PBS-Tween 0,1%-BSA 1%, 45 mn à 37°C), chaque AcM, préparé aux concentrations de 0,1 - 1 et 10 µg/ml, a été incubé 1 heure à 37°C.

Après cinq lavages, un anticorps secondaire (anti-IgM de souris couplé à la peroxydase, Jackson Laboratories) a été incubé 1h à 37°C (100 µl/puits). Après cinq lavages, la peroxydase est révélée avec du TMB (100 µl/puits), 30 mn à température ambiante puis arrêtée avec H₂SO₄ 1M (50 µl/puits).

L'intensité de la réaction colorée est quantifiée (DO) et va servir de référence pour calculer le pourcentage de réaction croisée pour chaque hormone testée. Pour les LH comme pour les FSH, la valeur de DO mesurée avec l'hormone ovine de référence est considérée comme la valeur 100%. Le pourcentage de réaction croisée est le rapport entre DO hormone testée et DO hormone ovine X 100.

### 1) Réaction croisée avec les LH d'origine porcine, bovine et avec la gonadotrophine chorionique humaine (hCG)

Les trois AcM reconnaissent les LH porcine et bovine avec un pourcentage de réaction croisée égal ou supérieur à la oLH, et croisent également avec l'hormone humaine hCG (voir Tableau X ci-dessous).

**Tableau X :**

| | B6 | D3 | G11 |
|---|---|---|---|
| oLH | 100% | 100% | 100% |
| pLH | 168% | 170% | 140% |
| bLH | 122% | 121% | 100% |
| hCG | 97% | 72% | 68% |

### 2) Réaction croisée avec les FSH d'origine porcine, bovine et humaine

Les trois AcM reconnaissent les FSH porcine, bovine et humaine avec un pourcentage de réaction croisée égal ou supérieur à la oFSH (voir Tableau XI ci-dessous).

**Tableau XI :**

| | B6 | D3 | G11 |
|---|---|---|---|
| oFSH | 100% | 100% | 100% |
| pFSH | 137% | 100% | 230% |
| bFSH | 430% | 460% | 380% |
| hFSH | 100% | 100% | 113% |

Les trois AcM présentent donc un profil de spécificité semblable, caractérisé par un large spectre de reconnaissance en faveur des hormones homologues porcines, bovines et humaines.

### 3) Réaction croisée avec la choriogonadotropine équine (eCG) ou PMSG

De la même façon, les anticorps ont été évalués sur de la eCG commerciale (Synchro Part, CE VA, Libourne, France) utilisée dans les traitements d'induction de l'ovulation chez les ovins et caprins. Un témoin isotypique (IgM dirigé contre un autre type d'antigène très éloigné des hormones gonadotropes). Chaque anticorps a été incubé à la concentration de 10µg/ml et 1µg/ml. Les résultats, exprimés en unités de densité optique (DO), sont illustrés dans le Tableau XII ci-dessous.

**Tableau XII**

| DO sur eCG commerciale | B6 | D3 | G11 | Anticorps contrôle |
|---|---|---|---|---|
| Anticorps à 10µg/ml | 0,088 | 0,091 | 0,09 | 0,09 |
| Anticorps à 1µg/ml | 0,078 | 0,102 | 0,088 | 0,09 |

Aucune réaction croisée avec la eCG commerciale n'est observée : la valeur de DO est la même que ce soit avec les anticorps spécifiques ou avec le témoin isotypique.

Les mêmes résultats ont été obtenus en évaluant les anticorps sur de la eCG purifée (6000 UI/mg).

### EXEMPLE 10: POLYMORPHISME DES FRAMEWORKS 1 DES ACM B6, D3 ET G11

Les trois AcM B6, D3 et G11 ont des séquences de CDR1, CDR2 et CDR3 identiques pour leur chaîne légère comme pour leur chaîne lourde.

De même, les séquences des frameworks FR2, FR3 et FR4 de leurs chaînes VH et VL sont identiques.

Seules les séquences des FR1 des VH et VL varient selon l'AcM.

### 1) FR1 de la chaîne légère (VL) :

Un polymorphisme est observé dans les résidus en position 4 et 7 du FR1 de la chaîne légère (voir Tableau XIII ci-dessous).

**Tableau XIII :**

| | FR1 (acides aminés 1 à 10 de VL) |
|---|---|
| B6 | DIV**M**TQ**A**TSS (SEQ ID NO : 20) |
| D3 | --- **K**TQ**T**TSS (SEQ ID NO : 22) |
| G11 | DIQ**M**TQ**T**TSS (SEQ ID NO : 18) |

En position 4, on note une méthionine (M), acide aminé à chaîne latérale non polaire hydrophobe, ou une lysine (K), acide aminé à chaîne latérale chargée positivement. Ce polymorphisme induit un changement de propriétés physico-chimiques important, du fait de la présence ou non d'une charge positive, mais ne constitue pas un élément structural commun aux deux AcM potentialisant la bioactivité de la LH et celle de la FSH.

En position 7, on note une alanine (A), acide aminé à chaîne non polaire hydrophobe, ou une thréonine (T), acide aminé à chaîne polaire non chargée. Il s'agit dans ce cas d'un polymorphisme relativement conservateur des propriétés physico-chimiques des acides aminés. Là encore, cela ne constitue pas un élément structural commun aux deux AcM potentialisant la bioactivité de la LH et celle de la FSH.

Donc, dans la chaîne légère, aucun polymorphisme du FR1 ne semble associé au double effet potentialisant LH et FSH.

### 2) FR1 de la chaîne lourde (VH) :

Un polymorphisme est observé au niveau des résidus en position 3 et 6 du FR1 de la chaîne lourde (voir Tableau XIV ci-dessous).

**Tableau XIV :**

| | FR1 (acides aminés 1 à 10 de VH) |
|---|---|
| B6 | EV**Q**LQ**Q**SGAE (SEQ ID NO : 21) |
| D3 | EV**Q**LQ**E**SGAE (SEQ ID NO : 23) |
| G11 | EV**K**LQ**Q**SGAE (SEQ ID NO : 19) |

En position 3, on note une glutamine (Q), acide aminé à chaîne polaire non chargée, ou une lysine (K), acide aminé à chaîne latérale chargée positivement. Il s'agit là d'un polymorphisme induisant un changement important dans les propriétés physico-chimiques de cette région. La présence d'une glutamine en position 3 apparaît spécifique des deux AcM potentialisant la bioactivité de la LH et celle de la FSH.

En position 6, on note une glutamine (Q), acide aminé à chaîne polaire non chargée, ou un acide glutamique (E), acide aminé à chaîne latérale chargée négativement. Là encore, il s'agit d'un polymorphisme induisant un changement important des propriétés physico-chimiques, mais qui dans ce cas ne paraît pas associé au double effet potentialisant LH et FSH.

### EXEMPLE 11: EFFET DE L'ANTICORPS D3 ET DU SCFV B6 SUR LA PRODUCTION DE PROGESTÉRONE PAR LES CELLULES MLTC STIMULÉES PAR L'OLH OU L'HCG.

Les cellules sont cultivées en milieu RPMI 1640 additionné de 10% FBS et 1% pénicilline/streptomycine. Elles sont sevrées 1 heure avant la stimulation par l'hormone (oLH ou hCG) seule ou le complexe anticorps/hormone ou scFv/hormone.

### Stimulation par oLH :

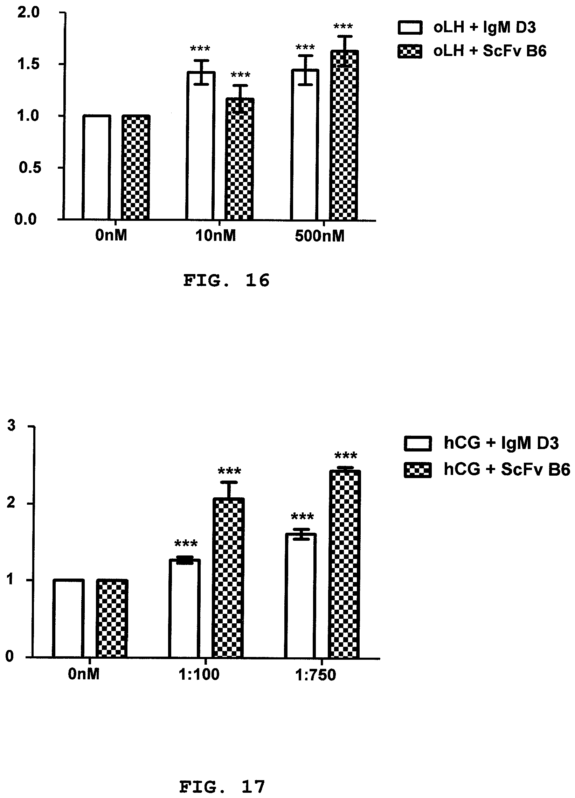

La quantité de progestérone sécrétée par les cellules MLTC stimulées avec 0,5nM d'oLH seule, a été comparée avec celle sécrétée par les cellules MLTC stimulées avec 0,5nM d'oLH préincubée avec l'IgM D3 entière ou le scFv B6, à 10nM ou 500nM. Les résultats sont illustrés sur la Figure 16. En abscisse sont indiquées les concentrations en IgM ou scFv (0 nM pour la stimulation avec oLH seule) ; en ordonnée, le rapport de la quantité de progestérone sécrétée en présence du complexe D3/oLH ou B6/oLH à la quantité de progestérone sécrétée en présence d'oLH seule. Aux deux concentrations, le scFv B6 et l'IgM D3 exercent un effet potentialisant significatif sur la réponse stéroïdogène des cellules MLTC, par rapport à une stimulation avec l'oLH seule (*p<0,001* par test de Bonferroni). L'effet potentialisant est maximal dès la concentration de 10nM d'IgM D3 et à la concentration de 500nM pour le scFv B6.

### Stimulation par hCG :

La même expérience a été réalisée avec hCG à la concentration constante de 0,05nM. La quantité de progestérone sécrétée a été comparée dans le cas d'une stimulation avec 0,05nM de hCG seule et dans le cas d'une stimulation avec le complexe hCG 0,05nM + IgM D3 entière ou hCG 0,05nM + scFv B6, à 5nM et 37.5nM. Les résultats sont illustrés sur la Figure 17. En abscisse sont indiquées les concentrations en IgM ou scFv (0 nM pour la stimulation avec hCG seule) ; en ordonnée, le rapport de la quantité de progestérone sécrétée en présence du complexe D3/hCG ou scFv B6/hCG à la quantité de progestérone sécrétée en présence d'hCG seule. Aux deux concentrations, le scFv B6 et l'IgM D3 exercent un effet potentialisant significatif sur la réponse stéroïdogène des cellules MLTC, par rapport à une stimulation avec l'hCG seule (*p<0,001* par test de Bonferroni). L'effet potentialisant est maximal à la concentration de 37.5nM d'IgM D3 ou de scFv B6.

### EXEMPLE 12 : EFFET POTENTIALISANT IN VIVO D'UN DIABODY DÉRIVÉ DE L'ANTICORPS B6 B5P0 SUR L'ACTIVITÉ FSH CHEZ LA RATTE

Un diabody, dénommé ci-après B5P0, a été construit à partir de la séquence des VH et VL de l'anticorps B6, reliées par un lieur de 5 acides aminés.

Les séquences nucléotidique et peptidique de ce diabody sont respectivement représentées dans la liste de séquences en annexe sous les numéros SEQ ID NO: 29 et SEQ ID NO: 30.

L'effet potentialisant du diabody B5P0 sur l'activité FSH *in vivo* a été déterminé par le test de Steelman et Pohley chez la ratte immature, comme décrit à l'Exemple 7 ci-dessus.

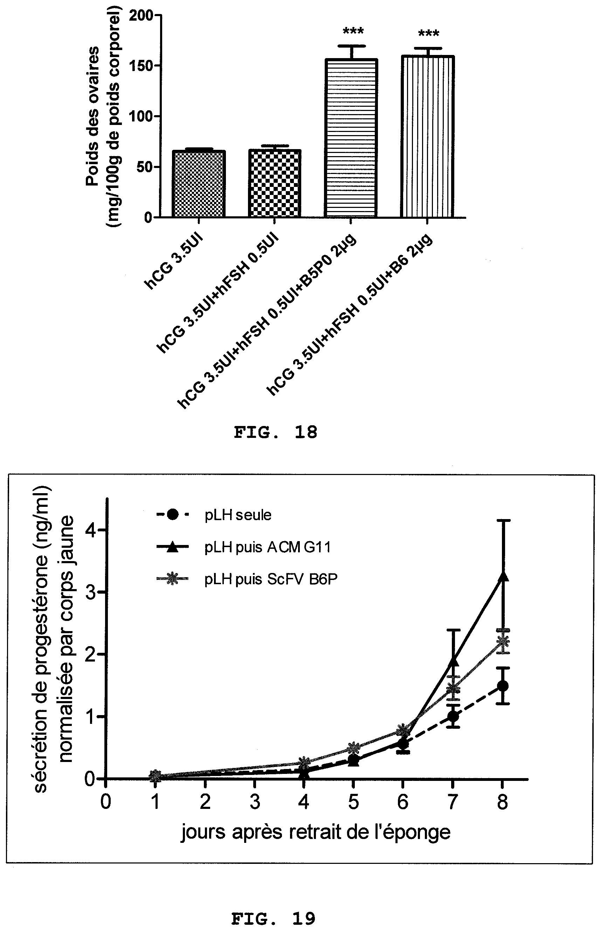

Les résultats sont illustrés par la Figure 18.

Ces résultats montrent que le diabody B5P0 (2µg) préalablement incubé avec 0.5UI de hFSH exerce un effet potentialisant équivalent à celui de l'IgM B6 entière (2µg). Cet effet entraîne dans les deux cas une augmentation du poids des ovaires d'un facteur 2.1, hautement significative (p<0.001, test de Bonferroni).

### EXEMPLE 13: EFFET POTENTIALISANT DU SCFV B6 IN VIVO SUR LES ACTIVITÉS LH ET FSH CHEZ LA BREBIS

### a) Effet potentialisant du scFv B6 sur l'activité LH

L'étude a été réalisée sur des brebis Ile de France, pubères, âgées de 3 ans. L'effet potentialisant du scFv B6 sur l'activité de la LH endogène a été évalué en comparaison avec celui de l'IgM entière G11.

Les brebis ont toutes été synchronisées par la pose d'une éponge vaginale imprégnée d'un progestagène (45 mg d'acétate de fluorogestone (FGA) - Intervet - France) pendant 14 jours.

36 heures après le retrait de l'éponge, les animaux ont reçu une injection de 3mg de pLH par voie intra-veineuse. Les brebis ont été réparties en trois lots, A, B, et C :
* lot A : traitées avec pLH seule
* lot B : traitées avec pLH puis scFv B6 ;
* lot C : traitées avec pLH puis IgM G11.

72 heures après le retrait de l'éponge, les animaux des lots B et C ont reçu respectivement, 2mg de scFv B6 purifié ou 2mg de l'IgM G11 purifiée, par voie intra-musculaire.

Des prises de sang quotidiennes sont effectuées du premier jour au 8^{ème} jour après retrait de l'éponge pour doser la progestérone plasmatique. 8 jours après retrait de l'éponge, des endoscopies sont effectuées pour compter et dater les corps jaunes

Les résultats d'endoscopie sont illustrés dans le Tableau XV.

**Tableau XV**

| Traitement | Nombre de corps jaunes | Datation des corps jaunes |
|---|---|---|
| Lot A : pLH seule 3mg | 2,09 ± 3,23 | 4,06 ± 4,7 |
| Lot B : pLH puis scFv B6P 2mg | 1,33 ± 0,58 | 5 ± 0,5 |
| Lot C : pLH puis IgM G11 2m | 2 ± 0,71 | 5,2 ± 0,27 |

Le nombre de corps jaunes est exprimé en moyenne ± écart-type. Il n'y a pas de différence significative entre le nombre de corps jaunes obtenus dans les trois lots [analyse par test T (GraphPad PRISM Software; GraphPad, San Diego, CA)]. La datation des corps jaunes est exprimée en nombre de jours post-ovulation (moyenne ± écart-type). L'âge moyen des corps jaunes est de 5 jours pour les lots traités avec l'IgM G11 ou le scFv B6P contre un âge moyen de 4 jours pour le lot traité avec la pLH seule. Cette différence d'âge des corps jaunes entre le lot A et les lots B et C est significative (p<0,05). Il n'y a pas de différence significative entre les lots B et C.Ces résultats signifient que chez les brebis traitées avec la pLH puis l'IgM ou le scFv, l'ovulation a eu lieu 1 jour avant celle observée chez les brebis traitées par la pLH seule. Le scFv B6 exerce donc le même effet potentialisant, *in vivo* chez la brebis, que l'anticorps entier G11.

Le profil de sécrétion de la progestérone en début de phase lutéale est représenté sur la Figure 19.

Pour chaque lot, les valeurs de concentration en progestérone (ng/ml), ont été normalisées par nombre de corps jaunes. Chaque courbe représente la moyenne des valeurs de progestérone obtenue chez les femelles de chaque lot. Les profils de sécrétion en P4 ont été comparés par analyse de variances à deux variables (*two-way* ANOVA, GraphPad PRISM Software; GraphPad, San Diego, CA). Cette analyse a montré que la courbe de sécrétion de P4, moyenne, est significativement différente entre le lot A et les lots B et C (p>0,05) et qu'il n'y a pas de différence entre les courbes du lot B et C.Ces résultats indiquent que le scFv B6 comme l'AcM G11, injecté seul, exerce un effet potentialisant, se manifestant par la mise en place d'une sécrétion de progestérone plus importante et plus rapide que dans le lot traité avec pLH seule. Ce résultat est corrélé à la précocité du moment de l'ovulation observée par endoscopie chez les brebis traitées avec pLH puis scFv B6P ou G11.

L'ensemble de ces résultats indique que le scFv B6 est capable de se lier à la LH ovine endogène, et de potentialiser son activité *in vivo,* avec la même efficacité que l'ACM entier G11.

### b) Effet potentialisant du scFv B6 sur l'activité FSH

L'étude a été réalisée en période de repos sexuel (jours longs) et a porté sur 18 brebis âgées de 4 ans. Les brebis ont toutes été synchronisées préalablement aux protocoles, par la pose d'une éponge vaginale imprégnée d'un progestagène (45 mg d'acétate de fluorogestone (FGA) - Intervet - France) pendant 14 jours.

24 heures et 12 heures avant le retrait de l'éponge, les brebis ont reçu des injections de 100µg puis de 83µg de pFSH pure par voie intra-musculaire

Les brebis ont été réparties en deux lots :
* lot A : traitées avec pFSH seule
* lot B : traitées avec pFSH puis scFv B6.

Les brebis du lot B ont reçu, par voie intra-musculaire, 3 injections successives de 1mg de scFv B6P purifié : la première à J0 lors du retrait de l'éponge, la seconde à J1, et la troisième à J3.

A J7: des endoscopies ont été réalisées pour compter le nombre de corps jaunes.

Une mesure du pic préovulatoire de LH a été réalisée par un dosage ELISA quantitatif pour toutes les femelles.

Les résultats d'endoscopie et de dosage de LH sont illustrés dans le Tableau XVI :

**Tableau XVI**

| Traitement | Nombre de brebis ayant ovulé | Nombre de corps jaunes chez les brebis ayant ovulé | Datation du pic de LH en heures après retrait de l'éponge |
|---|---|---|---|
| Lot A : pFSH seule | 1/7 | 6 | 54 |
| Lot B : pFSH puis scFv B6P | 3/7 | 23 | 48 |
| | | 16 | 48 |
| | | 3 | 48 |

On observe que le nombre de femelles ayant ovulé est significativement plus important dans le lot traité par pFSH puis scFv B6P (3/7 versus 1/7). Le lot B présente également un nombre d'ovulations significativement plus important [(p< 0,001), analyse par test T (GraphPad PRISM Software; GraphPad, San Diego, CA)] Par ailleurs, dans le lot pFSH puis scFv B6P, les trois femelles ayant ovulé ont eu un pic de LH synchrone, à 48 heures après retrait de l'éponge, et avancé de 12 heures par rapport à celle du lot pFSH seule. Cette différence est significative (p<0,005).

Ces résultats indiquent que, compte tenu de la demi-vie courte de la pFSH (30 minutes), le scFv B6P a induit un effet potentialisant de la FSH endogène traduit par un nombre d'ovulations plus élevé et un pic de LH très synchrone.

## Revendications

1. Anticorps monoclonal, ou anticorps recombinant ou fragment d'anticorps dérivé de celui-ci et liant l'antigène, capable de se lier à une hormone lutéinisante (LH), **caractérisé en ce que** le domaine variable de la chaîne lourde contient les CDRs suivants :
- VH-CDR1, défini par la séquence GYTFTNYW (SEQ ID NO: 13);
- VH-CDR2, défini par la séquence IYPGGGYT (SEQ ID NO: 14);
- VH-CDR3, défini par la séquence ARTPLYGSSYGGFAY (SEQ ID NO: 15); et le domaine variable de la chaîne légère contient les CDRs suivants :
- VL-CDR1, défini par la séquence QGISNY (SEQ ID NO: 16);
- VL-CDR2, défini par la séquence YTS;
- VL-CDR3, défini par la séquence QQYSKLPWT (SEQ ID NO: 17).

2. Anticorps monoclonal, ou anticorps recombinant ou fragment d'anticorps dérivé de celui-ci et liant l'antigène selon la revendication 1, **caractérisé en ce que** la portion N-terminale de la région framework FR1 de sa chaine lourde est définie par la séquence X₁VQLQX₁SGAE (SEQ ID NO : 24) dans laquelle X₁ représente une glutamine ou un acide glutamique.

3. Anticorps monoclonal, ou anticorps recombinant ou fragment d'anticorps dérivé de celui-ci et liant l'antigène selon la revendication 2, **caractérisé en ce que** la portion N-terminale de la région framework FR1 de sa chaine lourde est définie par la séquence SEQ ID NO : 24 dans laquelle X₁ représente une glutamine.

4. Anticorps monoclonal, ou anticorps recombinant ou fragment d'anticorps dérivé de celui-ci et liant l'antigène selon une quelconque des revendications 2 ou 3, **caractérisé en ce que** la portion N-terminale de la région FR1 de la chaine légère dudit ligand contient la séquence X₂TQX₃TSS (SEQ ID NO : 25), dans laquelle X₂ représente une méthionine ou une lysine et X₃ représente une thréonine ou une alanine.

5. Anticorps monoclonal, ou anticorps recombinant ou fragment d'anticorps dérivé de celui-ci et liant l'antigène selon la revendication 2, choisi parmi:
a) l'anticorps monoclonal 1A6 C4 G11 produit par l'hybridome CNCM I-4332 ;
b) un fragment Fab, Fab', ou Fab'2 de l'anticorps a), ci-dessus ;

6. Anticorps monoclonal, ou anticorps recombinant ou fragment d'anticorps dérivé de celui-ci et liant l'antigène selon la revendication 3, choisi parmi:
a) l'anticorps monoclonal 9A4 A7 D3 produit par l'hybridome CNCM I-4333 ;
b) l'anticorps monoclonal 9A4 D4 B6 produit par l'hybridome CNCM I-4334 ;
c) un fragment Fab, Fab', ou Fab'2 d'un anticorps a) ou b) ci-dessus.

7. Anticorps monoclonal, ou anticorps recombinant ou fragment d'anticorps dérivé de celui-ci et liant l'antigène selon l'une quelconque des revendications 1 à 6, pour son utilisation comme médicament.

8. Anticorps monoclonal, ou anticorps recombinant ou fragment d'anticorps dérivé de celui-ci et liant l'antigène selon l'une quelconque des revendications 1 à 7, pour potentialiser la bioactivité de la LH.

9. Anticorps monoclonal, ou anticorps recombinant ou fragment d'anticorps dérivé de celui-ci et liant l'antigène selon l'une quelconque des revendications 1, 2, 3, 4, 6, ou 7, pour potentialiser la bioactivité de la LH et la bioactivité de la FSH.

10. Complexe anticorps -gonadotrophine choisi parmi :
- un complexe d'un anticorps monoclonal, ou anticorps recombinant ou fragment d'anticorps dérivé de celui-ci et liant l'antigène selon une quelconque des revendications 1 à 6 avec la LH ou la hCG ;
- un complexe d'un anticorps monoclonal, ou anticorps recombinant ou fragment d'anticorps dérivé de celui-ci et liant l'antigène selon une quelconque des revendications 1, 2, 3, 4, et 6 avec la FSH.

11. Complexe selon la revendication 10, pour son utilisation comme médicament.

12. Anticorps monoclonal, ou anticorps recombinant ou fragment d'anticorps dérivé de celui-ci et liant l'antigène selon une quelconque des revendications 1 à 9 ou complexe selon une quelconque des revendications 10 ou 11, pour induire l'ovulation chez un mammifère femelle.

## Patentansprüche

1. Monoklonaler Antikörper oder rekombinanter Antikörper oder Antikörperfragment, das davon abgeleitet ist und das Antigen bindet, der in der Lage ist, sich mit einem luteinisierenden Hormon (LH) zu binden, **dadurch gekennzeichnet, dass** der variable Bereich der schweren Kette die folgenden CDRs enthält:
- VH-CDR1, definiert durch die Sequenz GYTFTNYW (SEQ ID NO:13);
- VH-CDR2, definiert durch die Sequenz IYPGGGYT (SEQ ID NO:14);
- VH-CDR3, definiert durch die Sequenz ARTPLYGSSYGGFAY (SEQ ID NO:15); und
der variable Bereich der leichten Kette die folgenden CDRs enthält:
- VL-CDR1, definiert durch die Sequenz QGISNY (SEQ ID NO:16);
- VL-CDR2, definiert durch die Sequenz YTS;
- VL-CDR3, definiert durch die Sequenz QQYSKLPWT (SEQ ID NO:17).

2. Monoklonaler Antikörper oder rekombinanter Antikörper oder Antikörperfragment, das davon abgeleitet ist und das Antigen bindet, nach Anspruch 1, **dadurch gekennzeichnet, dass** der N-terminale Abschnitt der Framework-Region FR1 seiner schweren Kette durch die Sequenz X₁VQLQX₁SGAE (SEQ ID NO:24) definiert ist, in der X₁ ein Glutamin oder eine Glutaminsäure darstellt.

3. Monoklonaler Antikörper oder rekombinanter Antikörper oder Antikörperfragment, das davon abgeleitet ist und das Antigen bindet, nach Anspruch 2, **dadurch gekennzeichnet, dass** der N-terminale Abschnitt der Framework-Region FR1 seiner schweren Kette durch die Sequenz SEQ ID NO:24 definiert ist, in der X₁ ein Glutamin darstellt.

4. Monoklonaler Antikörper oder rekombinanter Antikörper oder Antikörperfragment, das davon abgeleitet ist und das Antigen bindet, nach einem der Ansprüche 2 oder 3, **dadurch gekennzeichnet, dass** der N-terminale Abschnitt der Region FR1 der leichten Kette des Liganden die Sequenz X₂TQX₃TSS (SEQ ID NO:25) enthält, in der X₂ ein Methionin oder ein Lysin darstellt und X₃ ein Threonin oder ein Alanin darstellt.

5. Monoklonaler Antikörper oder rekombinanter Antikörper oder Antikörperfragment, das davon abgeleitet ist und das Antigen bindet, nach Anspruch 2, der ausgewählt ist aus:
a) dem monoklonalen Antikörper 1A6 C4 G11, der durch das Hybridom CNCM I-4332 erzeugt wird;
b) einem Fab-, Fab'- oder Fab'2-Fragment des vorhergehenden Antikörpers a).

6. Monoklonaler Antikörper oder rekombinanter Antikörper oder Antikörperfragment, das davon abgeleitet ist und das Antigen bindet, nach Anspruch 3, der ausgewählt ist aus:
a) dem monoklonalen Antikörper 9A4 A7 D3, der durch das Hybridom CNCM I-4333 erzeugt wird;
b) dem monoklonalen Antikörper 9A4 D4 B6, der durch das Hybridom CNCM I-4334 erzeugt wird;
c) einem Fab-, Fab'- oder Fab'2-Fragment von einem der vorhergehenden Antikörper a) oder b).

7. Monoklonaler Antikörper oder rekombinanter Antikörper oder Antikörperfragment, das davon abgeleitet ist und das Antigen bindet, nach einem der Ansprüche 1 bis 6 für seine Verwendung als Medikament.

8. Monoklonaler Antikörper oder rekombinanter Antikörper oder Antikörperfragment, das davon abgeleitet ist und das Antigen bindet, nach einem der Ansprüche 1 bis 7, zur Potenzierung der Bioaktivität des LH.

9. Monoklonaler Antikörper oder rekombinanter Antikörper oder Antikörperfragment, das davon abgeleitet ist und das Antigen bindet, nach einem der Ansprüche 1, 2, 3, 4, 6 oder 7 zur Potenzierung der Bioaktivität des LH und der Bioaktivität des FSH.

10. Antikörper-Gonadotropin-Komplex, der ausgewählt ist aus:
- einem Komplex eines monoklonalen Antikörpers oder rekombinanten Antikörpers oder Antikörperfragments, das davon abgeleitet ist und das Antigen bindet, nach einem der Ansprüche 1 bis 6 mit dem LH oder dem hCG;
- einem Komplex eines monoklonalen Antikörpers oder rekombinanten Antikörpers oder Antikörperfragments, das davon abgeleitet ist und das Antigen bindet, nach einem der Ansprüche 1, 2, 3, 4 und 6 mit dem FSH.

11. Komplex nach Anspruch 10 für seine Verwendung als Medikament.

12. Monoklonaler Antikörper oder rekombinanter Antikörper oder Antikörperfragment, das davon abgeleitet ist und das Antigen bindet, nach einem der Ansprüche 1 bis 9 oder Komplex nach einem der Ansprüche 10 oder 11 zum Induzieren der Ovulation bei einem weiblichen Säugetier.

## Claims

1. A monoclonal antibody, or recombinant antibody or antibody fragment derived therefrom and binding the antigen, capable of binding to a luteinizing hormone (LH), **characterized in that** the variable domain of the heavy chain contains the following CDRs:
- VH-CDR1, defined by the sequence GYTFTNYW (SEQ ID NO: 13) ;
- VH-CDR2, defined by the sequence IYPGGGYT (SEQ ID NO: 14)
- VH-CDR3, defined by the sequence ARTPLYGSSYGGFAY
(SEQ ID NO: 15); and the variable domain of the lightweight chain contains the following CDRs:
- VL-CDR1, defined by the sequence QGISNY (SEQ ID NO: 16) ;
- VL-CDR2, defined by the sequence YTS;
- VL-CDR3, defined by the sequence QQYSKLPWT(SEQ ID NO: 17).

2. The monoclonal antibody, or recombinant antibody or antibody fragment derived therefrom and binding the antigen, according to claim 1, **characterized in that** the N-terminal portion of the framework region FR1 of its heavy chain is defined by the sequence X₁VQLQX₁SGAE (SEQ ID NO: 24) wherein X₁ represents glutamine or glutamic acid.

3. The monoclonal antibody, or recombinant antibody or antibody fragment derived therefrom and binding the antigen, according to claim 2, **characterized in that** the N-terminal portion of the framework region FR1 of its heavy chain is defined by the sequence SEQ ID NO: 24 wherein X₁ represents glutamine.

4. The monoclonal antibody, or recombinant antibody or antibody fragment derived therefrom and binding the antigen, according to any of claims 2 or 3, **characterized in that** the N-terminal portion of the region FR1 of its lightweight chain of said ligand contains the sequence X₂TQX₃TSS (SEQ ID NO: 25) wherein X₂ represents a methionine or lysine and X₃ represents a threonine or alanine.

5. The monoclonal antibody, or recombinant antibody or antibody fragment derived therefrom and binding the antigen, according to claim 2, selected from among:
a) the monoclonal antibody 1A6C4G11 produced by the hybridoma CNCM 1-4332;
b) a fragment Fab, Fab', or Fab'2 of the antibody a), above.

6. The monoclonal antibody, or recombinant antibody or antibody fragment derived therefrom and binding the antigen, according to claim 3, selected from among:
a) the monoclonal antibody 9A4A7D3 produced by the hybridoma CNCM 1-4333;
b) the monoclonal antibody 9A4D4B6 produced by the hybridoma CNCM 1-4334;
c) a fragment Fab, Fab', or Fab'2 of an antibody a) or b), above.

7. The monoclonal antibody, or recombinant antibody or antibody fragment derived therefrom and binding the antigen, according to any of claims 1 to 6, for its use as a drug.

8. The monoclonal antibody, or recombinant antibody or antibody fragment derived therefrom and binding the antigen, according to any of claims 1 to 7, for potentializing the bioactivity of the LH.

9. The monoclonal antibody, or recombinant antibody or antibody fragment derived therefrom and binding the antigen, according to any of claims 1, 2, 3, 4, 6, or 7, for potentializing the bioactivity of the LH and the bioactivity of the FSH.

10. An antibody-gonatropin complexe selected from among:
- a complex of a monoclonal antibody, or recombinant antibody or antibody fragment derived therefrom and binding the antigen, according to any of claims 1 to 6, with the LH or the hCG;
- a complex of a monoclonal antibody, or recombinant antibody or antibody fragment derived therefrom and binding the antigen, according to any of claims 1, 2, 3, 4, and 6, with the FSH.

11. The complex according to claim 10, for its use as drug.

12. The monoclonal antibody, or recombinant antibody or antibody fragment derived therefrom and binding the antigen, according to any of claims 1 to 9 or complex according to any of claims 10 or 11, for inducing ovulation in a female mammal.


### SEQUENCE LISTING

<110> INSTITUT NATIONAL DE LA RECHERCHE AGRONOMIQUE MAUREL, Marie-Christine REITER, Eric GUILLOU, Florian
<120> Ligand d'hormone lutéinisante
<130> F539-147PCT
<150> FR 10 04503
   <151> 2010-11-19
<160> 30
<170> PatentIn version 3.5
<210> 1
   <211> 368
   <212> DNA
   <213> Mus musculus
<400> 1
   gaggtccaac tgcaggagtc aggagctgag ctggtaaggc ctgggacttc agtgaagata      60
   tcctgcaagg cttctggcta caccttcact aactactggc taggttgggt aaagcagagg     120
   cctggacatg gacttgagtg gattggagat atttaccctg gaggtggtta tactaactac     180
   aatgagaagt tcaagggcaa ggccacactg actgcagaca catcctccag cactgcctac     240
   atgcagctca gtagcctgac atctgaggac tctgctgtct atttctgtgc aagaacccct     300
   ctctacggta gtagctacgg ggggtttgct tactggggcc aagggactct ggtcactgtc     360
   tctgcaga                                                              368
<210> 2
   <211> 122
   <212> PRT
   <213> Mus musculus
<220>
   <221> MISC_FEATURE
   <222> (26)..(33)
   <223> résidus constituant le VH-CDR1
<220>
   <221> MISC_FEATURE
   <222> (51)..(58)
   <223> résidus constituant le VH-CDR2
<220>
   <221> MISC_FEATURE
   <222> (97)..(111)
   <223> résidus constituant le VH-CDR3
<400> 2
   Glu Val Gln Leu Gln Glu Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Thr Pro Leu Tyr Gly Ser Ser Tyr Gly Gly Phe Ala Tyr Trp
               100                 105                 110
   Gly Gln Gly Thr Leu Val Thr Val Ser Ala
           115                 120
<210> 3
   <211> 313
   <212> DNA
   <213> Mus musculus
<400> 3
   aagacacaga ctacatcctc cctgtctgcc tctctgggag acagagtcac catcagttgc       60
   agtgcaagtc agggcattag caattattta aactggtatc agcagaaacc agatggaact      120
   gttaaactcc tgatctatta cacatcaagt ttacactcag gagtcccatc aaggttcagt      180
   ggcagtgggt ctgggacaga ttattctctc accatcagca acctggaacc tgaagatatt      240
   gccacttact attgtcagca gtatagtaag cttccgtgga cgttcggtgg aggcaccaag      300
   ctggaaatca aac                                                         313
<210> 4
   <211> 104
   <212> PRT
   <213> Mus musculus
<220>
   <221> MISC_FEATURE
   <222> (24) .. (29)
   <223> résidus constituant le VL-CDR1
<220>
   <221> MISC_FEATURE
   <222> (47)..(49)
   <223> résidus constituant le VL-CDR2
<220>
   <221> MISC_FEATURE
   <222> (86)..(94)
   <223> résidus constituant le VL-CDR3
<400> 4
   Lys Thr Gln Thr Thr Ser Ser Leu Ser Ala Ser Leu Gly Asp Arg Val
   1               5                   10                  15
   Thr Ile Ser Cys Ser Ala Ser Gln Gly Ile Ser Asn Tyr Leu Asn Trp
               20                  25                  30
   Tyr Gln Gln Lys Pro Asp Gly Thr Val Lys Leu Leu Ile Tyr Tyr Thr
           35                  40                  45
   Ser Ser Leu His Ser Gly Val Pro Ser Arg Phe Ser Gly Ser Gly Ser
       50                  55                  60
   Gly Thr Asp Tyr Ser Leu Thr Ile Ser Asn Leu Glu Pro Glu Asp Ile
   65                  70                  75                  80
   Ala Thr Tyr Tyr Cys Gln Gln Tyr Ser Lys Leu Pro Trp Thr Phe Gly
                   85                  90                  95
   Gly Gly Thr Lys Leu Glu Ile Lys
               100
<210> 5
   <211> 368
   <212> DNA
   <213> Mus musculus
<400> 5
   gaggtgaagc tgcagcagtc aggagctgag ctggtaaggc ctgggacttc agtgaagata    60
   tcctgcaagg cttctggcta caccttcact aactactggc taggttgggt aaagcagagg   120
   cctggacatg gacttgagtg gattggagat atttaccctg gaggtggtta tactaactac   180
   aatgagaagt tcaagggcaa ggccacactg actgcagaca catcctccag cactgcctac   240
   atgcagctca gtagcctgac atctgaggac tctgctgtct atttctgtgc aagaacccct   300
   ctctacggta gtagctacgg ggggtttgct tactggggcc aagggactct ggtcactgtc   360
   tctgcaga                                                            368
<210> 6
   <211> 122
   <212> PRT
   <213> Mus musculus
<220>
   <221> MISC_FEATURE
   <222> (26)..(33)
   <223> résidus constituant le VH-CDR1
<220>
   <221> MISC_FEATURE
   <222> (51)..(58)
   <223> résidus constituant le VH-CDR2
<220>
   <221> MISC_FEATURE
   <222> (97)..(111)
   <223> résidus constituant le VH-CDR3
<400> 6
   Glu Val Lys Leu Gln Gln Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Thr Pro Leu Tyr Gly Ser Ser Tyr Gly Gly Phe Ala Tyr Trp
               100                 105                 110
   Gly Gln Gly Thr Leu Val Thr Val Ser Ala
           115                 120
<210> 7
   <211> 322
   <212> DNA
   <213> Mus musculus
<400> 7
   gatatccaga tgacacagac tacatcctcc ctgtctgcct ctctgggaga cagagtcacc        60
   atcagttgca gtgcaagtca gggcattagc aattatttaa actggtatca gcagaaacca       120
   gatggaactg ttaaactcct gatctattac acatcaagtt tacactcagg agtcccatca       180
   aggttcagtg gcagtgggtc tgggacagat tattctctca ccatcagcaa cctggaacct      240
   gaagatattg ccacttacta ttgtcagcag tatagtaagc ttccgtggac gttcggtgga      300
   ggcaccaagc tggaaatcaa ac                                                322
<210> 8
   <211> 107
   <212> PRT
   <213> Mus musculus
<220>
   <221> MISC_FEATURE
   <222> (27)..(32)
   <223> résidus constituant le VL-CDR1
<220>
   <221> MISC_FEATURE
   <222> (50)..(52)
   <223> résidus constituant le VL-CDR2
<220>
   <221> MISC_FEATURE
   <222> (89)..(97)
   <223> résidus constituant le VL-CDR3
<400> 8
   Asp Ile Gln Met Thr Gln Thr Thr Ser Ser Leu Ser Ala Ser Leu Gly
   1               5                   10                  15
   Asp Arg Val Thr Ile Ser Cys Ser Ala Ser Gln Gly Ile Ser Asn Tyr
               20                  25                  30
   Leu Asn Trp Tyr Gln Gln Lys Pro Asp Gly Thr Val Lys Leu Leu Ile
           35                  40                  45
   Tyr Tyr Thr Ser Ser Leu His Ser Gly Val Pro Ser Arg Phe Ser Gly
       50                  55                  60
   Ser Gly Ser Gly Thr Asp Tyr Ser Leu Thr Ile Ser Asn Leu Glu Pro
   65                  70                  75                  80
   Glu Asp Ile Ala Thr Tyr Tyr Cys Gln Gln Tyr Ser Lys Leu Pro Trp
                   85                  90                  95
   Thr Phe Gly Gly Gly Thr Lys Leu Glu Ile Lys
               100                 105
<210> 9
   <211> 366
   <212> DNA
   <213> Mus musculus
<400> 9
   gaggtgcaac tgcagcagtc tggagctgag ctggtaaggc ctgggacttc agtgaagata      60
   tcctgcaagg cttctggcta caccttcact aactactggc taggttgggt aaagcagagg     120
   cctggacatg gacttgagtg gattggagat atttaccctg gaggtggtta tactaactac     180
   aatgagaagt tcaagggcaa ggccacactg actgcagaca catcctccag cactgcctac     240
   atgcagctca gtagcctgac atctgaggac tctgctgtct atttctgtgc aagaacccct     300
   ctctacggta gtagctacgg ggggtttgct tactggggcc aagggactct ggtcactgtc     360
   tctgca                                                                366
<210> 10
   <211> 122
   <212> PRT
   <213> Mus musculus
<220>
   <221> MISC_FEATURE
   <222> (26)..(33)
   <223> résidus constituant le VH-CDR1
<220>
   <221> MISC_FEATURE
   <222> (51)..(58)
   <223> résidus constituant le VH-CDR2
<220>
   <221> MISC_FEATURE
   <222> (97)..(111)
   <223> résidus constituant le VH-CDR3
<400> 10
   Glu Val Gln Leu Gln Gln Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Thr Pro Leu Tyr Gly Ser Ser Tyr Gly Gly Phe Ala Tyr Trp
               100                 105                 110
   Gly Gln Gly Thr Leu Val Thr Val Ser Ala
           115                 120
<210> 11
   <211> 321
   <212> DNA
   <213> Mus musculus
<400> 11
   gatattgtga tgacgcaggc tacatcctcc ctgtctgcct ctctgggaga cagagtcacc        60
   atcagttgca gtgcaagtca gggcattagc aattatttaa actggtatca gcagaaacca       120
   gatggaactg ttaaactcct gatctattac acatcaagtt tacactcagg agtcccatca       180
   aggttcagtg gcagtgggtc tgggacagat tattctctca ccatcagcaa cctggaacct       240
   gaagatattg ccacttacta ttgtcagcag tatagtaagc ttccgtggac gttcggtgga       300
   ggcaccaagc tggaaatcaa a                                                 321
<210> 12
   <211> 107
   <212> PRT
   <213> Mus musculus
<220>
   <221> MISC_FEATURE
   <222> (27)..(32)
   <223> résidus constituant le VL-CDR1
<220>
   <221> MISC_FEATURE
   <222> (50)..(52)
   <223> résidus constituant le VL-CDR2
<220>
   <221> MISC_FEATURE
   <222> (89)..(97)
   <223> résidus constituant le VL-CDR3
<400> 12
   Asp Ile Val Met Thr Gln Ala Thr Ser Ser Leu Ser Ala Ser Leu Gly
   1               5                   10                  15
   Asp Arg Val Thr Ile Ser Cys Ser Ala Ser Gln Gly Ile Ser Asn Tyr
               20                  25                  30
   Leu Asn Trp Tyr Gln Gln Lys Pro Asp Gly Thr Val Lys Leu Leu Ile
           35                  40                  45
   Tyr Tyr Thr Ser Ser Leu His Ser Gly Val Pro Ser Arg Phe Ser Gly
       50                  55                  60
   Ser Gly Ser Gly Thr Asp Tyr Ser Leu Thr Ile Ser Asn Leu Glu Pro
   65                  70                  75                  80
   Glu Asp Ile Ala Thr Tyr Tyr Cys Gln Gln Tyr Ser Lys Leu Pro Trp
                   85                  90                  95
   Thr Phe Gly Gly Gly Thr Lys Leu Glu Ile Lys
               100                 105
<210> 13
   <211> 8
   <212> PRT
   <213> Mus musculus
<400> 13
   Gly Tyr Thr Phe Thr Asn Tyr Trp
   1               5
<210> 14
   <211> 8
   <212> PRT
   <213> Mus musculus
<400> 14
   Ile Tyr Pro Gly Gly Gly Tyr Thr
   1 5
<210> 15
   <211> 15
   <212> PRT
   <213> Mus musculus
<400> 15
   Ala Arg Thr Pro Leu Tyr Gly Ser Ser Tyr Gly Gly Phe Ala Tyr
   1 5 10 15
<210> 16
   <211> 6
   <212> PRT
   <213> Mus musculus
<400> 16
   Gln Gly Ile Ser Asn Tyr
   1 5
<210> 17
   <211> 9
   <212> PRT
   <213> Mus musculus
<400> 17
   Gln Gln Tyr Ser Lys Leu Pro Trp Thr
   1 5
<210> 18
   <211> 10
   <212> PRT
   <213> Mus musculus
<400> 18
   Asp Ile Gln Met Thr Gln Thr Thr Ser Ser
   1 5 10
<210> 19
   <211> 10
   <212> PRT
   <213> Mus musculus
<400> 19
   Glu Val Lys Leu Gln Gln Ser Gly Ala Glu
   1 5 10
<210> 20
   <211> 10
   <212> PRT
   <213> Mus musculus
<400> 20
   Asp Ile Val Met Thr Gln Ala Thr Ser Ser
   1               5                   10
<210> 21
   <211> 10
   <212> PRT
   <213> Mus musculus
<400> 21
   Glu Val Gln Leu Gln Gln Ser Gly Ala Glu
   1               5                   10
<210> 22
   <211> 7
   <212> PRT
   <213> Mus musculus
<400> 22
   Lys Thr Gln Thr Thr Ser Ser
   1               5
<210> 23
   <211> 10
   <212> PRT
   <213> Mus musculus
<400> 23
   Glu Val Gln Leu Gln Glu Ser Gly Ala Glu
   1               5                   10
<210> 24
   <211> 10
   <212> PRT
   <213> Artificial Sequence
<220>
   <223> Séquence consensus
<220>
   <221> MISC_FEATURE
   <222> (1)..(1)
   <223> Xaa représente un résidu glutamine ou acide glutamique
<220>
   <221> MISC_FEATURE
   <222> (6) .. (6)
   <223> Xaa représente un résidu glutamine ou acide glutamique
<400> 24
   Xaa Val Gln Leu Gln Xaa Ser Gly Ala Glu
   1               5                   10
<210> 25
   <211> 7
   <212> PRT
   <213> Artificial Sequence
<220>
   <223> Séquence consensus
<220>
   <221> MISC_FEATURE
   <222> (1)..(1)
   <223> Xaa représente une méthionine ou une lysine
<220>
   <221> MISC_FEATURE
   <222> (4)..(4)
   <223> Xaa représente une thréonine ou une alanine
<400> 25
   Xaa Thr Gln Xaa Thr Ser Ser
   1               5
<210> 26
   <211> 10
   <212> PRT
   <213> Artificial Sequence
<220>
   <223> Séquence consensus
<220>
   <221> MISC_FEATURE
   <222> (3)..(3)
   <223> Xaa représente une glutamine ou une valine
<220>
   <221> MISC_FEATURE
   <222> (4)..(4)
   <223> Xaa représente une méthionine ou une lysine
<220>
   <221> MISC_FEATURE
   <222> (7)..(7)
   <223> Xaa représente une thréonine ou une alanine
<220>
   <221> MISC_FEATURE
   <222> (7)..(7)
   <223> Xaa représente une thréonine ou une alanine
<400> 26
   Asp Ile Xaa Xaa Thr Gln Xaa Thr Ser Ser
   1               5                   10
<210> 27
   <211> 10
   <212> PRT
   <213> Artificial Sequence
<220>
   <223> Séquence consensus
<400> 27
   Asp Ile Gln Met Thr Gln Ala Thr Ser Ser
   1               5                   10
<210> 28
   <211> 244
   <212> PRT
   <213> artificial sequence
<220>
   <223> scFv B6
<220>
   <221> MISC_FEATURE
   <222> (1)..(122)
   <223> domaine VH
<220>
   <221> MISC_FEATURE
   <222> (123)..(137)
   <223> lieur peptidique
<220>
   <221> MISC_FEATURE
   <222> (138)..(244)
   <223> domaine VL
<220>
   <221> MISC_FEATURE
   <222> (145)..(145)
   <223> Xaa représente un résidu thréonine ou un résidu proline
<400> 28
   Gln Val Gln Leu Gln Gln Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Thr Pro Leu Tyr Gly Ser Ser Tyr Gly Gly Phe Ala Tyr Trp
               100                 105                 110
   Gly Gln Gly Thr Leu Val Thr Val Ser Ala Gly Gly Gly Gly Ser Gly
           115                 120                 125
   Gly Gly Gly Ser Gly Gly Gly Gly Ser Asp Ile Gln Met Thr Gln Thr
       130                 135                 140
   Xaa Ser Ser Leu Ser Ala Ser Leu Gly Asp Arg Val Thr Ile Ser Cys
   145                 150                 155                 160
   Ser Ala Ser Gln Gly Ile Ser Asn Tyr Leu Asn Trp Tyr Gln Gln Lys
                   165                 170                 175
   Pro Asp Gly Thr Val Lys Leu Leu Ile Tyr Tyr Thr Ser Ser Leu His
               180                 185                 190
   Ser Gly Val Pro Ser Arg Phe Ser Gly Ser Gly Ser Gly Thr Asp Tyr
   195 200 205
   Ser Leu Thr Ile Ser Asn Leu Glu Pro Glu Asp Ile Ala Thr Tyr Tyr
   210 215 220
   Cys Gln Gln Tyr Ser Lys Leu Pro Trp Thr Phe Gly Gly Gly Thr Lys
   225 230 235 240
   Leu Glu Ile Lys
<210> 29
   <211> 711
   <212> DNA
   <213> Artificial sequence
<220>
   <223> Diabody
<220>
   <221> CDS
   <222> (1)..(705)
<400> 29
   cag gtt cag ctg cag cag agc ggt gca gaa ctg gtt cgt ccg ggt aca     48
   Gln Val Gln Leu Gln Gln Ser Gly Ala Glu Leu Val Arg Pro Gly Thr
   1               5                   10                  15
   agc gtt aaa att agc tgt aaa gcc agc ggc tat acc ttt acc aat tat     96
   Ser Val Lys Ile Ser Cys Lys Ala Ser Gly Tyr Thr Phe Thr Asn Tyr
               20                  25                  30
   tgg ctg ggt tgg gtt aaa cag cgt ccg ggt cat ggt ctg gaa tgg att     144
   Trp Leu Gly Trp Val Lys Gln Arg Pro Gly His Gly Leu Glu Trp Ile
           35                  40                  45
   ggt gat att tat ccg ggt ggt ggc tat acc aat tat aat gaa aaa ttt     192
   Gly Asp Ile Tyr Pro Gly Gly Gly Tyr Thr Asn Tyr Asn Glu Lys Phe
       50                  55                  60
   aaa ggc aaa gca acc ctg acc gca gat acc agc agc agc acc gca tat     240
   Lys Gly Lys Ala Thr Leu Thr Ala Asp Thr Ser Ser Ser Thr Ala Tyr
   65                  70                  75                  80
   atg cag ctg tct agc ctg acc agc gaa gat agc gca gtt tat ttc tgt     288
   Met Gln Leu Ser Ser Leu Thr Ser Glu Asp Ser Ala Val Tyr Phe Cys
                   85                  90                  95
   gca cgc aca ccg ctg tat ggt agc agc tat ggt ggt ttt gca tat tgg     336
   Ala Arg Thr Pro Leu Tyr Gly Ser Ser Tyr Gly Gly Phe Ala Tyr Trp
               100                 105                 110
   ggt cag ggc acc ctg gtt acc gtt agc gca ggt ggt ggt gga tcc gat     384
   Gly Gln Gly Thr Leu Val Thr Val Ser Ala Gly Gly Gly Gly Ser Asp
           115                 120                 125
   att cag atg acc cag acc ccg tct agc ctg agc gca agc ctg ggt gat     432
   Ile Gln Met Thr Gln Thr Pro Ser Ser Leu Ser Ala Ser Leu Gly Asp
       130                 135                 140
   cgt gtt acc att agc tgt agc gca agc cag ggt att agc aat tat ctg     480
   Arg Val Thr Ile Ser Cys Ser Ala Ser Gln Gly Ile Ser Asn Tyr Leu
   145                 150                 155                 160
   aat tgg tat cag cag aaa ccg gat ggc acc gtt aaa ctg ctg att tat     528
   Asn Trp Tyr Gln Gln Lys Pro Asp Gly Thr Val Lys Leu Leu Ile Tyr
                   165                 170                 175
   tat acc agc agc ctg cat agc ggt gtt ccg agc cgt ttt agc ggt agc     576
   Tyr Thr Ser Ser Leu His Ser Gly Val Pro Ser Arg Phe Ser Gly Ser
               180                 185                 190
   ggt tct ggc acc gat tat agc ctg acc att tct aat ctg gaa ccg gaa     624
   Gly Ser Gly Thr Asp Tyr Ser Leu Thr Ile Ser Asn Leu Glu Pro Glu
           195                 200                 205
   gat att gcc acc tat tat tgc cag cag tat agc aaa ctg ccg tgg acc     672
   Asp Ile Ala Thr Tyr Tyr Cys Gln Gln Tyr Ser Lys Leu Pro Trp Thr
       210                 215                 220
   ttt ggt ggt ggc acc aaa ctg gag att aaa taa ctcgag                  711
   Phe Gly Gly Gly Thr Lys Leu Glu Ile Lys
   225                 230
<210> 30
   <211> 234
   <212> PRT
   <213> Artificial sequence
<220>
   <223> Synthetic Construct
<400> 30
   Gln Val Gln Leu Gln Gln Ser Gly Ala Glu Leu Val Arg Pro Gly Thr
   1               5                   10                  15
   Ser Val Lys Ile Ser Cys Lys Ala Ser Gly Tyr Thr Phe Thr Asn Tyr
               20                  25                  30
   Trp Leu Gly Trp Val Lys Gln Arg Pro Gly His Gly Leu Glu Trp Ile
           35                  40                  45
   Gly Asp Ile Tyr Pro Gly Gly Gly Tyr Thr Asn Tyr Asn Glu Lys Phe
       50                  55                  60
   Lys Gly Lys Ala Thr Leu Thr Ala Asp Thr Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Thr Pro Leu Tyr Gly Ser Ser Tyr Gly Gly Phe Ala Tyr Trp
               100                 105                 110
   Gly Gln Gly Thr Leu Val Thr Val Ser Ala Gly Gly Gly Gly Ser Asp
           115                 120                 125
   Ile Gln Met Thr Gln Thr Pro Ser Ser Leu Ser Ala Ser Leu Gly Asp
       130                 135                 140
   Arg Val Thr Ile Ser Cys Ser Ala Ser Gln Gly Ile Ser Asn Tyr Leu
   145                 150                 155                 160
   Asn Trp Tyr Gln Gln Lys Pro Asp Gly Thr Val Lys Leu Leu Ile Tyr
                   165                 170                 175
   Tyr Thr Ser Ser Leu His Ser Gly Val Pro Ser Arg Phe Ser Gly Ser
               180                 185                 190
   Gly Ser Gly Thr Asp Tyr Ser Leu Thr Ile Ser Asn Leu Glu Pro Glu
           195                 200                 205
   Asp Ile Ala Thr Tyr Tyr Cys Gln Gln Tyr Ser Lys Leu Pro Trp Thr
       210                 215                 220
   Phe Gly Gly Gly Thr Lys Leu Glu Ile Lys
   225                 230